# EUROPEAN PATENT APPLICATION

(11) **EP 4 679 940 A2**
(43) Date of publication of application: **14.01.2026**
(21) Application number: 25216497.5
(22) Date of filing: 08.08.2022
(51) Int. Cl.: H05H 1/24

(54) **PLASMA TREATMENT APPARATUS**

(30) Priority: 17.09.2021 KR 20210124944; 17.09.2021 KR 20210124946; 17.09.2021 KR 20210124952; 29.10.2021 KR 20210146784; 23.12.2021 KR 20210186633; 24.12.2021 KR 20210186977; 28.01.2022 KR 20220013567; 26.04.2022 KR 20220051210; 29.04.2022 KR 20220053605; 25.05.2022 KR 20220064367
(62) Divisional of application: 22805746.9
(71) Applicant: Plasmapp Co., Ltd., Yuseong-gu Daejeon 34141 (KR)
(72) Inventor: LIM, You Bong, 34141 Yuseong-gu Daejeon (KR)
(74) Representative: Jung, Minkyu

(57) **Abstract**

Provided is a plasma treatment apparatus that does not use a separate process gas by discharging low-pressure atmosphere formed in a sealed space in which a plasma surface treatment is performed. Therefore, by discharging the low-pressure atmosphere using a relatively low voltage, stable plasma with high surface treatment efficiency may be generated, and thus a plasma treatment apparatus may be configured economically and no operation and management costs due to the use of a process gas are incurred. Also, the safety and the effectiveness of a plasma surface treatment process to be applied in the medical industry are secured, and sterility may be secured and usability may be maximized.

## Description

### Technical Field

The disclosure relates to a plasma treatment apparatus, and more particularly, to a plasma treatment apparatus for imparting characteristics according to plasma treatment to an object to be treated without using a separate process gas.

### Background Art

Plasma is an ionized gas containing free electrons, ionized atoms and molecules, radicals, etc. When a large energy is applied to a gas from among states of a material, the material becomes a plasma state including electrons and ions, which are different from those in a general phase transition. Since plasma contains high-energy charged particles, it actively reacts with other materials and exhibit physical and chemical changes, and thus plasma is used in various industries.

According to conventional plasma technology used in the industries, plasma is generated by using a high frequency or the like in a high vacuum. Surface treatment technology using plasma in such a vacuum environment has been used as a core manufacturing process technology in major high-tech industries such as semiconductors, displays, and solar cells since the 1980s, researches on vacuum plasma technology have been actively conducted for the purposes like deposition, etching, ashing, etc.

According to a conventional plasma treatment technology using such a vacuum plasma, an object to be treated is introduced into a vacuum chamber, and a sufficient vacuum (e.g., 1 mTorr or less) is formed by using a high vacuum pump to increase the pressure inside the vacuum chamber. By sufficiently securing a vacuum inside the vacuum chamber and injecting a process gas, surface treatment according to plasma discharge and the characteristics of the process gas become possible. Here, the primary purpose of securing a sufficient degree of vacuum is to sufficiently remove atmospheric gases to increase the purity of a subsequently injected process gas, and the process gas is selected according to a purpose like deposition, etching, and ashing, wherein plasma surface treatment efficiency is secured by adjusting a mixture, an injection amount, and an exhaust rate of injected gases according to the purpose of the surface treatment.

Atmospheric pressure plasma technology does not use an expensive vacuum pump, thus having an advantage that plasma treatment may be performed without configuring a separate sealed chamber environment. According to the atmospheric pressure plasma technology, in the same regard as the vacuum plasma technology, a process gas is selected according to the purpose of a surface treatment to obtain a targeted surface treatment effect. However, atmospheric pressure plasma technology has a limitation in that it is difficult to control the partial pressure of a highly-pure process gas, because the process gas is injected in an atmospheric environment and treatment is performed in an environment in which atmospheric molecules are mixed. Also, since atmospheric pressure plasma technology is processed in an open space rather than a sealed vacuum chamber, types of gases that may be used are limited due to the problem of exposing a process gas to the outside, and exposure of by-products such as active species generated through plasma discharge. In particular, due to atmospheric gases (nitrogen and oxygen) that cannot be excluded from a plasma generation environment in the atmospheric environment, there is a risk in securing user safety against exposure to reactive nitrogen species (RNS) and reactive oxygen species (ROS) generated by plasma reaction.

The atmosphere generally refers to a gas that surrounds the entire perimeter uniformly and is a gas that surrounds the Earth due to the Earth's gravity. The constituent gases of the atmosphere include nitrogen (typically about 78%) and oxygen (typically about 21%), as well as argon (typically about 0.93%), carbon dioxide, neon, helium, etc. The atmosphere is a mixture of various gases and is distributed in a constant composition up to a certain altitude (generally about 80 km from the surface of the Earth) except for carbon dioxide and ozone due to the movement of the air. The partial pressure refers to the pressure of each of components of a mixed gas like the atmosphere. For example, in the atmosphere of 1 atm, the partial pressure of nitrogen is 0.78 atm, and the partial pressure of oxygen is 0.21 atm.

In the conventional atmospheric pressure plasma technology, a surface treatment technique using an inert gas (e.g., helium or argon, etc.) is widely used to improve plasma stability while minimizing by-products such as active species generated in the atmosphere. Although it may be less economical to perform plasma surface treatment while continuously supplying a large amount of gas as described above, the technique features relatively high economic competitiveness as compared to techniques requiring an expensive vacuum chamber, a vacuum pump, and precise supply control of high purity for implementation of the conventional vacuum plasma technology. Therefore, atmospheric pressure plasma techniques are being used in application industries that are relatively less sensitive in terms of precision (secondary battery, textile, environment, etc.). Representatively, in the agricultural field, researches and developments are being conducted to sterilize and promote germination of seeds, and researches and developments are also conducted to improve the preservation of food through non-heating sterilization of food using plasma.

Recently, the atmospheric pressure plasma techniques have been used in various ways in the medical industry, and there is a technique for sterilization of a medical device or a technique for cleaning impurities remaining on the surface of a medical device. In particular, in the case of medical implants, which are medical devices for bio-insertion, various materials have been developed to improve bio-compatibility, and there are surface treatment techniques to increase the effective surface area of a surface, wherein the effective surface area of a bio material may be increased and the inflammatory reaction caused by impurities may be reduced by cleaning the impurities on the surface of the bio material. Also, plasma treatment is used for various purposes such as control of surface energy (crosslinking reaction, structural change by surface chemical reaction, sterilization, wettability, adhesion, bonding, surface strengthening, enhancement of surface heat resistance, etc.).

There is a technique used for the purpose of removing impurities and improving surface energy by using atmospheric pressure plasma, wherein the technique has a process mechanism similar to that of an ashing process technique using oxygen gas in industrial plasma.

Industrial plasma ashing process technique is typically used for the purpose of cleaning and removing photoresist remaining after a process or by-products generated in a previous process during a semiconductor manufacturing process. The ashing process uses a process gas containing an oxygen-containing gas to generate oxygen active species and the like to remove by-products generated in a semiconductor manufacturing process (Prior Document Korean Patent No. 10-1226297), and, by employing the technical configuration of the Prior Document, a technique for treating the surface of a dental implant by using oxygen plasma modified from an oxygen-containing gas has been developed (Prior Document: Korean Open Patent No. 10-2016-0065698).

An ashing process of semiconductors using industrial plasma and a technique for surface treatment of medical implants using atmospheric pressure plasma in the medical industry remove organic substances such as hydrocarbons (CHx) through plasma treatment, and plasma reaction in which active species (OH) are generated for sterilization and surface energy improvement may be simply expressed as follows.

CHₓ + O₂ + (Plasma)→CO₂ + H₂O + OH

To improve the performance of medical implants used in the medical industry, the medical implant manufacturing process includes a surface treatment process to increase the effective surface area of a surface, a cleaning process to remove impurities, and a sterilization process to ensure sterility. However, even when impurities are completely removed during the manufacturing process, surface contamination occurring during the conventional sterilization process (ethylene oxide (EO) gas or radiation), the distribution stage, and the storage stage is an unavoidable phenomenon.

Conventional plasma technology used in the medical industry obtains a surface treatment effect through removal of impurities (e.g., hydrocarbons) and improvement of surface energy (formation of OH functional groups on a surface of an implant) by using atmospheric pressure plasma. For the primary purpose of reducing by-products caused by plasma generation, inert gases such as argon and helium are used as process gases. Also, by injecting such process gases, the voltage for plasma generation may be lowered and plasma may be stably generated on a surface of an implant, thereby preventing damage to the surface due to plasma. However, although the actual process gas used to remove impurities or increase surface energy in the surface reaction formula is oxygen, since a separate process gas is injected to reduce by-products to reduce the partial pressure of oxygen, the treatment efficiency is deteriorated.

The efficiency of the atmospheric pressure plasma technology is not only limited due to the partial pressure of a process gas, but also in terms of energy of plasma discharged at high pressure. Plasma forms an electric field in a certain space, transfers energy through electrons that are accelerated above the electric field, and the accelerated electrons collide with surrounding process gas molecules and are ionized. Since The atmospheric pressure has a higher molecular density than that of a vacuum environment, a mean free path and an acceleration distance are relatively short, and thus plasma energy is low as compared to applied power. In other words, since impurities attached to a surface of an implant are attached with various energy values, when atmospheric pressure plasma is used, there is a limit in that only impurities attached with relatively low energy values may be removed.

The conventional atmospheric pressure plasma treatment techniques used in the medical industry may secure some user safety by using a process gas, but the safety level is not sufficient. Also, the conventional atmospheric pressure plasma treatment techniques have limits in plasma treatment efficiency and require a separate gas storage and a separate gas supply for using a process gas. Therefore, as components for operating and managing the gas storage and the gas supply are required, a high cost is incurred for introduction and operation of the gas storage and the gas supply and the usability is deteriorated.

Plasma technology has the effect of improving the efficiency of osseointegration, a functional connection between living bone and artificial implants, and further improving bio-compatibility by removing impurities from a surface of an object to be treated and improving surface energy thereof. Therefore, plasma technology may be used for surface treatment of dental implants, orthopedic implants, bone grafts, skin grafts, ophthalmic implants, cardiac implants, cochlear implants, cosmetic implants, nerve implants, etc. However, the medical industry is managing medical devices to be inserted into the human body, such as implants, as high-risk medical devices with the strictest requirements for sterility. Therefore, in order to apply plasma technology to the medical industry, surface treatment needs to be performed while maintaining the sterility of an object to be treated, and operation of a plasma treatment apparatus that does not cause secondary contamination of medical devices including the object to be treated is required.

Also, since the environment in which a plasma treatment apparatus is operated exposes patients who are vulnerable to gastrotoxic substances, it is very important to secure user safety against exposure of active species generated by plasma reaction.

Also, since the medical industry requires systematic verification of high safety and effectiveness for the introduction of new technologies due to the characteristics directly related to human life, a technique that enables simple, intuitive, and easy recognition of a verified result is required. Such a technique may have an improved effect in terms of not only safety and effectiveness, but also a user's usability.

In summary, the conventional plasma treatment technique using vacuum plasma generates stable plasma and achieves surface treatment efficiency by forming a high vacuum and using a highly pure process gas, but an expensive apparatus configuration is required therefor.

The conventional plasma treatment technology using atmospheric pressure plasma generates a stable plasma using an inert process gas in an atmospheric pressure environment and enables an economical configuration of a plasma treatment apparatus. However, there are limits including limited surface treatment efficiency and low safety due to a large amount of discharge by-products.

Also, since all of the conventional techniques use process gases, costs for operation and management thereof are incurred and usability is poor. In particular, the conventional techniques do not satisfy the safety and the effectiveness required to be applied to the medical industry, and usability thereof is limited.

### PRIOR ART DOCUMENTS

### [PATENT DOCUMENTS]

(Patent Document 0001) Japanese Patent Laid-Open No. 2002-313775
(Patent Document 0002) Korean Patent No. 10-2312813
(Patent Document 0003) Korean Patent No. 10-1226297
(Patent Document 0004) Korea Patent Publication No. 10-2016-0065698

### Description of Embodiments

### Technical Problem

The disclosure provides a plasma treatment apparatus for generating stable plasma by discharging low-pressure atmosphere formed by exhausting the atmosphere of a sealed space by using a relatively low voltage without using a separate process gas.

The disclosure also provides a plasma treatment apparatus of which the efficiency of surface treatments including removal of impurities from a surface of an object to be treated through plasma generation by discharging low-pressure atmosphere is improved through pressure control.

The disclosure also provides a plasma treatment apparatus with surface treatment efficiency improved by generating plasma by intensively discharging low-pressure atmosphere around an object to be treated through an electrode structure and voltage control.

The disclosure also provides a plasma treatment apparatus that generates plasma by using low-pressure atmosphere without using a separate process gas for reduced costs for operation and management and high usability.

The disclosure also provides a plasma treatment apparatus with improves user safety by reducing generation of by-products such as active species generated through plasma discharge by discharging low-pressure atmosphere.

The disclosure also provides a plasma treatment apparatus capable of treating an object to be treated while maintaining the sterility of the object to be treated by generating plasma around the object to be treated inside a packaged container.

The disclosure also provides a plasma treatment apparatus with improved usability and preventing secondary contamination of medical devices including objects to be treated by reducing user intervention in a surface treatment process.

The disclosure also provides a plasma treatment apparatus with improved usability as well as improved safety and effectiveness by allowing a user to simply and intuitively recognize a plasma surface treatment process.

In addition, the technical goals to be achieved by the disclosure are not limited to the technical goals mentioned above, and other technical goals may be clearly understood by one of ordinary skill in the art from the following descriptions.

### Solution to Problem

According to an aspect of the disclosure, there is provided a plasma treatment apparatus that does not use a separate process gas by discharging low-pressure atmosphere formed in a sealed space in which plasma surface treatment is to be performed. In this case, the plasma treatment apparatus does not include a separate process gas storage unit and a process gas supply unit, because a separate process gas is not used.

### Advantageous Effects of Disclosure

A plasma treatment apparatus according to the disclosure discharges low-pressure atmosphere formed by exhausting the atmosphere of a closed space by using a relatively low voltage without using a separate process gas, and thus stable plasma may be generated without damaging an object to be treated.

Also, the plasma treatment apparatus according to the disclosure may significantly improve the efficiency of surface treatments including removal of impurities from a surface of an object to be treated through plasma generation by discharging low-pressure atmosphere through pressure control.

Also, the plasma treatment apparatus according to the disclosure may improve surface treatment efficiency improved by generating plasma by intensively discharging low-pressure atmosphere around an object to be treated through an electrode structure and voltage control.

Also, since the plasma treatment apparatus according to the disclosure generates plasma by using low-pressure atmosphere without using a separate process gas, operation and management costs due to the use of process gases including the cost for an expensive gas flow controller are not incurred, and thus a pump and power supply may be economically configured, thereby enabling configuration of a plasma treatment apparatus with high economic efficiency and significantly improved usability.

Also, since the plasma treatment apparatus according to the disclosure discharges low-pressure atmosphere, generation of by-products such as active species generated through plasma discharge may be reduced. Also, by purifying some of by-products in a controllable fluid flow, user safety may be significantly improved.

Also, the plasma treatment apparatus according to the disclosure may maintain the sterility of an object to be treated during a surface treatment process by generating plasma around the object to be treated inside a packaged container.

Also, the plasma treatment apparatus according to the disclosure has an accommodation structure that reduces user intervention in the process of accommodating and discharging an object to be treated during a surface treatment process, thereby preventing secondary contamination of medical devices including the object to be treated and improving usability.

And, the plasma treatment apparatus according to the disclosure is configured such that a plasma surface treatment process may be visually recognized from the outside. Therefore, a user may check the operation of the plasma treatment apparatus simply and intuitively, and thus not only safety and effectiveness but also usability may be improved.

The effects obtainable in the example embodiments of the disclosure are not limited to the above-mentioned effects, and other effects not mentioned above may be clearly derived and understood by one of ordinary skill in the art from the following descriptions.

### Brief Description of Drawings

FIG. 1 is a block diagram showing a plasma treatment apparatus of the disclosure.
FIG. 2 is a conceptual diagram showing changes in the atmosphere inside a sealed unit of the plasma treatment apparatus of FIG. 1.
FIG. 3A shows a result of a numerical analysis of electron temperature and density of plasma expressed as a function of discharge pressure, and FIG. 3B shows a result of a numerical analysis of effective electron densities for respective critical values of energy expressed as a function of discharge pressure.
FIG. 4 is a diagram for describing the configuration of the plasma treatment apparatus of FIG. 1.
FIGS. 5A to 5D are diagrams for describing other embodiments of the sealed unit of the plasma treatment apparatus of FIG. 4.
FIGS. 6A to 6D are diagrams for describing other embodiments of the electrode unit of the plasma treatment apparatus of FIG. 4.
FIGS. 7A to 7F are block diagrams showing a plasma treatment apparatus according to an embodiment of the disclosure.
FIG. 8 is a flowchart for describing the process operation of the plasma treatment apparatus of FIG. 1.
FIGS. 9A to 9E are flowcharts for describing others embodiment of FIG. 8.
FIG. 10 is a graph showing changes in the internal pressure of the sealed unit of the plasma treatment apparatus of FIG. 1 over time.
FIGS. 11A to 11I are graphs showing other embodiments in which the internal pressure of the sealed unit of FIG. 10 changes over time.
FIGS. 12A to 12C are graphs showing power applied to the electrode unit of the plasma treatment apparatus of FIG. 1.
FIG. 13 is a perspective view of a plasma treatment apparatus according to an embodiment of the disclosure.

### Best Mode

According to an aspect of the disclosure, there is provided a plasma treatment apparatus that does not use a separate process gas by discharging low-pressure atmosphere formed in a sealed space in which plasma surface treatment is to be performed. In this case, the plasma treatment apparatus does not include a separate process gas storage unit and a process gas supply unit, because a separate process gas is not used.

Also, according to an aspect of the disclosure, the surface of an object to be treated is treated by generating oxygen and nitrogen active species through a plasma reaction using nitrogen and oxygen in low-pressure (or low-density) atmosphere.

According to an embodiment, the plasma treatment apparatus may include a sealed unit having an interior sealed against the external environment; a pressure adjusting unit for adjusting the internal pressure of the sealed unit within a pre-set process pressure range; and an electrode unit that forms an electric field inside the sealed unit.

According to an embodiment, the plasma treatment apparatus may further include a control unit that controls the pressure adjusting unit to form low-pressure atmosphere within the pre-set process pressure range inside the sealed unit by exhausting internal atmosphere of the sealed unit and controls the electrode unit to discharge the low-pressure atmosphere.

According to an embodiment, the pre-set process pressure range may be set in a range of 1 Torr or higher and less than 100 Torr.

According to an embodiment, the sealed unit may include an upper member and a lower member separated from each other, and the upper member or the lower member may move in relation to each other, thereby sealing the inside of the sealed unit.

According to an embodiment, the lower member may include a accommodating hole corresponding to the shape of an object to be treated or a container having accommodated therein the object to be treated, and the electrode unit may include an electrode disposed on an inner circumferential surface of the accommodating hole.

According to an embodiment, the inside of the sealed unit may be include a material having chemical resistance or a chemical resistant coating layer may be formed therein.

According to an embodiment, at least a portion of the sealed unit may include a transparent material, and thus the low-pressure atmosphere in the inside of the sealed unit in which plasma is discharged may be visually recognized from the outside.

According to an embodiment, the transparent material may be a glass material.

According to an embodiment, the electrode unit may include an electrode, and at least a portion of the electrode may be exposed into the inside of the sealed unit.

According to an embodiment, the electrode at least partially exposed into the inside of the sealed unit, may be electrically connected to an object to be treated accommodated in the sealed unit or a portion of a container having accommodated therein the object to be treated.

According to an embodiment, the electrode may be magnetic.

According to an embodiment, the pressure adjusting unit may include an exhaust unit for exhausting the inside of the sealed unit, and the control unit may control the exhaust unit to seal the inside of the sealed unit against the external environment.

According to an embodiment, the sealed unit may include an elastic member that is deformed, such that the inside of the sealed unit is sealed against the external environment by using a pressure difference between the inside and outside of the sealed unit.

According to an embodiment, the control unit may include a sensor for measuring a difference between a pressure of the inside and a pressure of the outside of the sealed unit.

According to an embodiment, the control unit may control the pressure adjusting unit to constantly maintain the internal pressure of the sealed unit within the pre-set process pressure range and discharge plasma inside the sealed unit in which the pressure of the low-pressure atmosphere is being maintained constant.

According to an embodiment, the pressure adjusting unit includes a vacuum pump for exhausting the internal atmosphere of the sealed unit, and the control unit may maintain the internal pressure of the sealed unit constant by continuously operating the vacuum pump and discharge plasma inside the sealed unit in which the pressure of the low-pressure atmosphere is maintained constant.

According to an embodiment, the pressure adjusting unit includes a vacuum pump for exhausting the internal atmosphere of the sealed unit and a valve for opening and closing a flow path interconnecting the sealed unit and the vacuum pump, and the control unit may maintain the internal pressure of the sealed unit constant by opening and closing the valve and discharge plasma inside the sealed unit in which the pressure of the low-pressure atmosphere is maintained constant.

According to an embodiment, the pressure adjusting unit includes a vacuum pump for exhausting the internal atmosphere of the sealed unit; a venting unit for injecting external atmosphere into the sealed unit; and a valve for opening and closing a flow path interconnecting the vacuum pump or the venting unit and the sealed unit, and the control unit may maintain the internal pressure of the sealed unit constant by opening and closing the valve and discharge plasma inside the sealed unit in which the pressure of the low-pressure atmosphere is maintained constant.

According to an embodiment, the pre-set process pressure range may be set in a range of 2 Torr or higher and less than 30 Torr.

According to an embodiment, the control unit may control the pressure adjusting unit to change the internal pressure of the sealed unit to be within the pre-set process pressure range and discharge plasma inside the sealed unit in which the pressure of the low-pressure atmosphere is being changed.

According to an embodiment, the pressure adjusting unit includes an exhaust unit for exhausting the internal atmosphere of the sealed unit and a valve for opening and closing a flow path interconnecting the sealed unit and the exhaust unit, and the control unit may change the internal pressure of the sealed unit by opening and closing the valve and discharge plasma inside the sealed unit in which the pressure of the low-pressure atmosphere is changed.

According to an embodiment, the pressure adjusting unit includes an exhaust unit for exhausting the internal atmosphere of the sealed unit; a venting unit for injecting external atmosphere into the sealed unit; and a valve for opening and closing a flow path interconnecting the exhaust unit or the venting unit and the sealed unit, and the control unit may change the internal pressure of the sealed unit by opening and closing the valve and discharge plasma inside the sealed unit in which the pressure of the low-pressure atmosphere is changed.

According to an embodiment, the control unit may control the pressure adjusting unit to repeatedly increase and decrease the internal pressure of the sealed unit, thereby discharging the low-pressure atmosphere of which the pressure is repeatedly changed inside the sealed unit.

According to an embodiment, the pressure adjusting unit may further include a filter that filters the external air injected into the sealed unit.

According to an embodiment, the control unit may include a sensor for measuring the internal pressure of the sealed unit.

According to an embodiment, the control unit may control the pressure adjusting unit based on a pre-set time.

According to an embodiment, the electrode unit may form the electric field with an alternating current (AC) power supply for a process time during which the internal pressure of the sealed unit is within the pre-set process pressure range and discharge the low-pressure atmosphere.

According to an embodiment, the controller may discharge the low-pressure atmosphere by maintaining the AC power supply ON during the process time.

According to an embodiment, the controller may discharge the low-pressure atmosphere by repeatedly turning the AC power supply ON and OFF during the process time.

According to an embodiment, the frequency of the AC power supply may be 10 kHz or higher and 200 kHz or lower.

According to an embodiment, the pressure adjusting unit includes an exhaust unit for exhausting the inside of the sealed unit, and the control unit may control the exhaust unit to remove by-products, which are generated by discharging the low-pressure atmosphere inside the sealed unit, from the inside of the sealed unit.

According to an embodiment, the exhaust unit may further include a filter for purifying the by-products.

According to an embodiment, the pressure adjusting unit includes a venting unit exposed to the external atmosphere and a valve for opening and closing a flow path interconnecting the sealed unit and the venting unit, and the control unit may form an atmospheric flow due to a pressure difference between the internal pressure of the sealed unit and the external atmosphere by opening the valve.

According to an embodiment, the control unit may control opening of the sealed unit by allowing the internal pressure of the sealed unit to be equalized to an external pressure by the atmospheric flow.

According to an embodiment, the object to be treated is accommodated inside the sealed unit and is surface-treated, and the object to be treated may be selected from a group consisting of dental implants, orthopedic implants, bone grafts, skin grafts, ophthalmic implants, heart implants, cochlear implants, cosmetic implants, nerve implants, medical resins, dental prostheses, fibers, seeds, and foods.

### Mode of Disclosure

Reference will now be made in detail to embodiments, examples of which are illustrated in the accompanying drawings. In the drawings, the same elements are denoted by the same reference numerals, and a repeated explanation thereof will not be given.

The disclosure may include various embodiments and modifications, and embodiments thereof will be illustrated in the drawings and will be described herein in detail. However, this is not intended to limit the disclosure to particular modes of practice, and it is to be appreciated that all changes, equivalents, and substitutes that do not depart from the spirit and technical scope of the disclosure are encompassed in the disclosure.

In the description of the disclosure, certain detailed explanations of the related art are omitted when it is deemed that they may unnecessarily obscure the essence of the disclosure. Also, numbers (for example, a first, a second, and the like) used in the description of the specification are merely identifier codes for distinguishing one element from another.

Also, in the present specification, it will be understood that when elements are "connected" or "coupled" to each other, the elements may be directly connected or coupled to each other, but may alternatively be connected or coupled to each other with an intervening element therebetween, unless specified otherwise.

Also, terms such as "~ unit", "~ group", "~ er(or)", and "~ module" used in this specification refer to a unit that processes at least one function or operation, wherein the unit may be implemented in the form of hardware like a processor, a microprocessor, micro controller, a central processing unit (CPU), a graphics processing unit (GPU), an accelerate processor unit (APU), a drive signal processor (DSP), an application specific integrated circuit (ASIC), a field programmable gate array (FPGA), etc., and may be implemented in a form to be combined with a memory that stores data necessary for processing at least one function or operation.

Also, it is intended to clarify that the components in the present specification are merely distinguished from one another according to main functions of the respective components. In other words, two or more components to be described below may be combined into one component, or one component may be divided into two or more components for more subdivided functions. In addition, each element described hereinafter may additionally perform some or all of functions performed by another element, in addition to main functions of itself, and some of the main functions of each element may be performed entirely by another component.

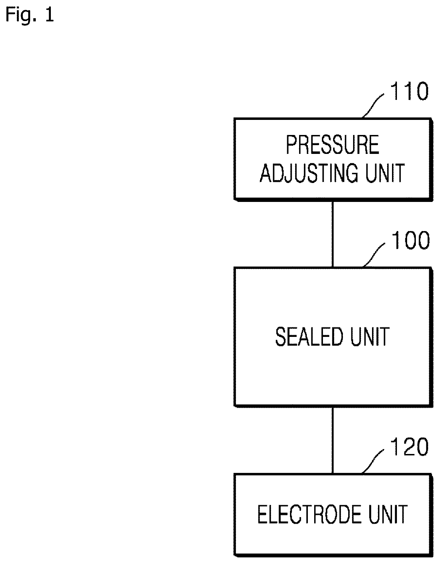

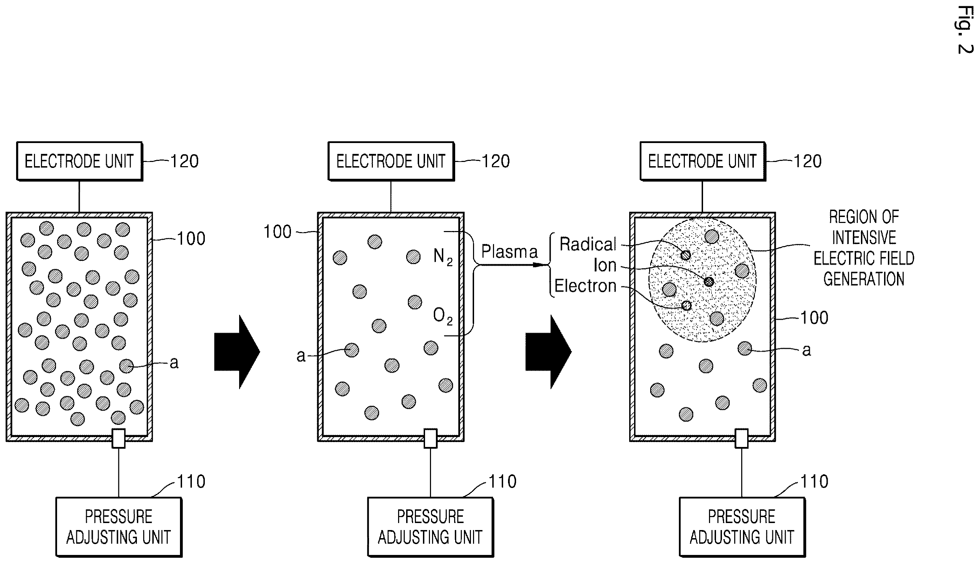

FIG. 1 is a block diagram showing a plasma treatment apparatus according to the disclosure, and FIG. 2 is a conceptual diagram showing changes in the atmosphere inside a sealed unit of the plasma treatment apparatus of FIG. 1.

Referring to FIGS. 1 and 2, the plasma treatment apparatus may include a sealed unit 100, a pressure adjusting unit 110, and an electrode unit 120.

The interior of the sealed unit 100 is sealed from the external environment. Therefore, the internal atmosphere of the sealed unit 100 is isolated from the external atmosphere and is composed of gas molecules (a) controllable by the plasma treatment apparatus.

The pressure adjusting unit 110 adjusts the internal pressure of the sealed unit 100 to a pre-set process pressure range. Therefore, a certain amount of the internal atmosphere of the sealed unit 100 is exhausted, and thus the internal atmosphere of the sealed unit 100 becomes a low-density atmosphere at a low-pressure within the pre-set process pressure range. At this time, the internal atmosphere (a) of the sealed unit 100 is distinguished from a gas environment formed by artificially modifying the gas composition ratio by using a process gas in conventional cases.

The electrode unit 120 forms an electric field inside the sealed unit 100. Therefore, the electrode unit 120 discharges the low-pressure atmosphere formed inside the sealed unit 100. Nitrogen molecules or oxygen molecules contained in the internal atmosphere of the sealed unit 100 are discharged by the electric field formed inside the sealed unit 100, and thus nitrogen active species or oxygen active species are generated. The electrode unit 120 or a member connected to the electrode unit 120 may control plasma generation by spatially forming a relatively electric field with high intensity inside the sealed unit 100 and place an object to be treated in an area corresponding to the plasma for surface treatment of the object to be treated. Through the discharge, gas molecules such as nitrogen or oxygen become ionized gas including free electrons, ionized atoms and molecules (ions), radicals (radicals), etc.

At this time, since the pressure of the internal atmosphere (a) of the sealed unit 100 is low, the internal atmosphere (a) of the sealed unit 100 contains nitrogen and oxygen in a low density, and thus plasma may be generated stably. Stable plasma is the form of discharge preventing damage to an object to be treated unlike plasma discharge that damages surfaces of an object to be treated due to a high current density like streamer discharge or filamentary discharge and may be, for example, glow discharge. Also, since the plasma treatment apparatus of the disclosure uses low-density nitrogen and oxygen, fewer discharge by-products are generated, and thus safety is improved.

Also, since the plasma treatment apparatus of the disclosure does not use a process gas in the plasma generation process, a separate process gas storage unit and a process gas injection unit are not needed. In other words, components for using a process gas are not needed, and thus an economical device configuration is possible. Also, the plasma treatment apparatus of the disclosure does not incur the cost of operating and managing a process gas, and the usability is also improved.

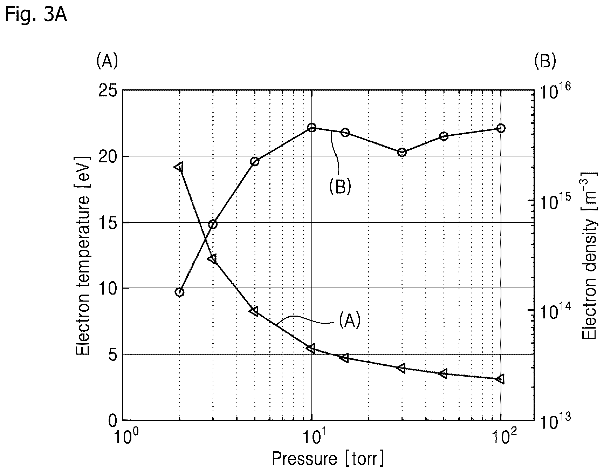

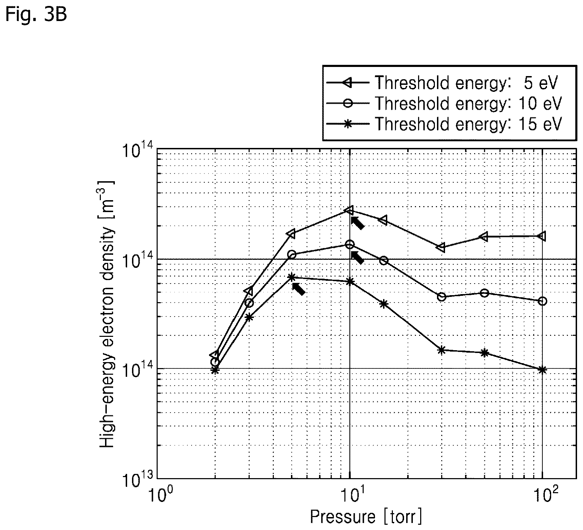

FIG. 3A shows a result of a numerical analysis of electron temperature and density of plasma expressed as a function of discharge pressure, and FIG. 3B shows a result of a numerical analysis of effective electron densities for respective critical values of energy expressed as a function of discharge pressure.

In detail, FIGS. 3A and 3B show results of evaluating the spatiotemporal behavior of electrons in plasma through numerical analyses. More specifically, a numerical analysis of electron temperature and density in order to find an optimal pressure condition for effectively removing hydrocarbon contaminants from surfaces of an implant is shown in FIG. 3A. As shown in FIG. 3A, as the pressure increases, the acceleration distance of electrons decreases, and thus the electron temperature (or energy of the electrons) decreases. On the other hand, as the pressure increases, the electron density increases. The increase in electron density according to the increase in pressure occurs in the pressure range up to about 10 Torr, and thereafter, the increase in the electron density is stalled. The critical value of energy for hydrocarbon dissociation is known to be about 20 electronvolts (eV), and methane, which is one of the hydrocarbon types with the simplest chemical structure, has the critical energy of 12.63eV. Methane is dissociated according to the following reaction equation.

CH₄ + e → CH₄⁺ + 2e

In this numerical analysis, it is assumed that electrons have a Boltzmann distribution. Therefore, the density of effective electrons may be estimated from the density of electrons having energy greater than a specific critical value by using an electron energy distribution function (EEDF). FIG. 3B shows the numerical analysis of the density of effective electrons having critical values of energy divided into 5 eV, 10 eV, and 15 eV, respectively. As indicated by the arrow in FIG. 3B, the density of effective electrons with energies of 5 eV or greater and that of effective electrons with energies of 10 eV or greater have maximum values at discharge pressures around 10 Torr, whereas the density of effective electrons with energies of 15 eV or greater has a maximum value at a discharge pressure around 5 Torr.

As a result, in order to generate more active electrons with energies of 15 eV or higher in terms of energy to dissociate bonds, plasma discharge at a pressure as low as 5 Torr is advantageous. Meanwhile, to increase the density of the entire effective electrons (or generation of active electrons having energy of 5 eV and/or 10 eV or greater), plasma discharge at a pressure around 10 Torr is more advantageous.

Also, a pressure of 2 Torr or less shows a sharp decrease in the density of effective electrons, thus being disadvantageous for hydrocarbon dissociation. Also, a pressure of 30 Torr or higher and less than 100 Torr shows a change in the density of stagnant effective electrons.

Also, plasma discharged in a pressure range of 100 Torr or higher is not preferable, because plasma characteristics or properties such as streamer discharge or filament discharge may be changed and damage an object to be treated. In particular, according to the Paschen's Curve according to Paschen's law, a high voltage needs to be applied for plasma discharge in a pressure range of 100 Toor or higher, and the temperature of an object to be treated increases due to plasma treatment.

Therefore, the pre-set process pressure range may be set in a range of 1 Torr or higher and less than 100 Torr. More preferably, the pre-set process pressure range may be set in a range of 2 Torr or higher and less than 30 Torr. Therefore, in the plasma treatment apparatus of the disclosure, it is possible to select an economical pump as compared to a plasma treatment apparatus using a vacuum plasma in which a pump having a very high degree of vacuum is used, and thus the economic efficiency of the configuration of the plasma treatment apparatus is improved.

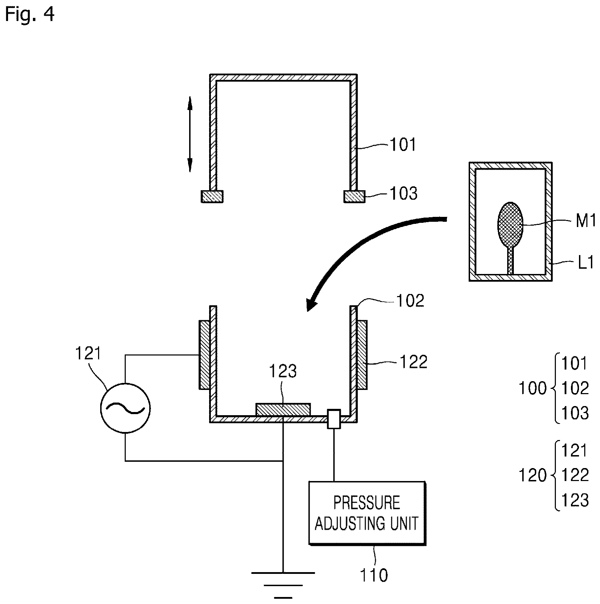

FIG. 4 is a diagram for describing the configuration of the plasma treatment apparatus of FIG. 1.

Referring to FIG. 4, the plasma treatment apparatus of the disclosure includes a sealed unit 100, which has an upper member 101, a lower member 102, and an elastic member 103, the pressure adjusting unit 110, and the electrode unit 120, which has a power supply unit 121, a first electrode 122, and a second electrode 123.

The sealed unit 100 seals the interior of the sealed unit 100 as the upper member 101 or the lower member 102 separated from each other move in relation to each other and contact each other. The sealed unit 100 may bring an object M1 to be treated or a container L1 in which the object M1 to be treated is accommodated into or out of the sealed unit 100 through an area opened as the upper member 101 and the lower member 102 are separated from each other by moving in relation to each other. Also, the sealed unit 100 seals the interior of the sealed unit 100 as the upper member 101 and the lower member 102 move in relation to each other and contact each other. Opening and sealing of the sealed unit 100 may be operated according to a user's input or information regarding accommodation of an object to be treated or completion of a processing process. Therefore, user convenience of the plasma treatment apparatus may be increased by minimizing a user's the intervention in the process of accommodation of an object to be treated, sealing for processing, and withdrawing of the object to be treated. In particular, when used in the medical industry including medical sites, safe medical services may be provided by reducing the risk of secondary contamination of medical devices including objects to be treated for treating a patient.

The object M1 to be treated is accommodated inside the sealed unit 100 and is surface-treated. The object M1 to be treated to be accommodated may include any object that is given characteristics according to plasma treatment and may be selected from a group consisting of dental implants, orthopedic implants, bone grafts, skin grafts, ophthalmic implants, heart implants, cochlear implants, cosmetic implants, nerve implants, medical resins, dental prostheses, fibers, seeds, and foods. Also, the object M1 to be treated may include a dental implant fixture, a dental abutment, a dental crown, an orthopedic stem, a cup, a cage, a spine, etc. may be a bone graft material that is a combination of at least one of synthetic bone, heterogenous bone, and allogeneic bone, and may be an electronic device for bio-insertion. Through a plasma surface treatment, the plasma treatment apparatus may form an oxide layer, such as a TiO₂ layer, on the surface of an object to be treated including Ti.

The object M1 to be treated may be accommodated inside the sealed unit 100 or the container L1 having accommodated or stored therein the object M1 to be treated may be accommodated inside the sealed unit 100. In the plasma treatment apparatus of the disclosure, the sterility of the object M1 to be treated may be maintained during a surface treatment process by accommodating the container L1, in which the object M1 to be treated is accommodated or stored, and surface-treating the object M1 to be treated. Also, the plasma treatment apparatus of the disclosure includes a plurality of sealed units 100, and the plurality of sealed units 100 may each be operated individually or may be operated simultaneously. To this end, the plasma treatment apparatus of the disclosure may include a plurality of pressure adjusting units 110 for individually or simultaneously adjusting the internal pressure of the sealed units 100 or a plurality of electrode units 120 for individually or simultaneously forming electric fields in the sealed units 100. At this time, the pressure adjusting unit 110 each includes one exhaust unit or a venting unit, and, by controlling a valve for opening and closing a flow path connected to each of the sealed units 100, the internal pressures of the sealed units 100 may be adjusted individually or simultaneously.

Alternatively, the sealed unit 100 may accommodate a plurality of objects to be treated or a container in which a plurality of objects to be treated are accommodated. Preferably, the plasma treatment apparatus of the disclosure may be configured to perform plasma surface treatment on two or more and five or less objects to be treated simultaneously or individually.

The lower member 102 may be fixed, and the upper member 101 may be connected to an elevating unit that elevates and lowers the upper member 101 in the vertical direction and move in relation to the lower member 102. Alternatively, the upper member 101 may be fixed, and the lower member 102 may be elevated and lowered. Alternatively, both the upper member 101 and the lower member 102 may move in relation to each other. Although it has been exemplified that relative movements are in vertical directions, the relative movements may include movements in other types of directions, including rotation.

The lower member 102 may include a accommodating hole corresponding to the shape of the object M1 to be treated or the container L1 having accommodated therein the object M1 to be treated. For example, the lower member 102 may have a cup-like shape with a hollow formed therein.

The lower member 102 may formed as a bottom member on which the object M1 to be treated or the container L1 having accommodated therein the object M1 to be treated is seated. In this case, the upper member 101 may include an outer circumferential surface corresponding to the shape of the object M1 to be treated or the container L1 having accommodated therein the object M1 to be treated.

The elastic member 103 is deformed, such that the inside of the sealed unit 100 is sealed against the external environment by using the pressure difference between the inside and the outside of the sealed unit 100. The elastic member 103 may include a flexible material such as silicone or rubber.

The elastic member 103 may be formed on a surface at which the upper member 101 and the lower member 102 contact each other. The elastic member 103 completes or enhances the sealing property of the upper member 101 and the lower member 102 to seal the interior of the sealing unit 10 against the external environment by being combined with each other. The elastic member 103 may be formed on the bottom surface of the upper member 101 or the top surface of the lower member 102. The elastic member 103 may have a hole through which internal air is exhausted or external air is injected to adjust the internal pressure of the sealed unit 100.

The sealed unit 100 may have any shape as long as the sealed unit 100 is capable of accommodating the object M1 to be treated or the container L1 having accommodated therein the object M1 to be treated. For example, the sealed unit 100 may have a cylindrical shape, a cuboidal shape, a hemispherical shape, etc. or may have an irregular shape.

Alternatively, the container L1 may be the sealed unit 100. The inside of the container L1 may be sealed against the external environment, and the internal pressure of the container L1 may be adjusted by the pressure adjusting unit 110. At this time, in the container L1, as the pressure adjusting unit 110 enters into the container L1, an exhaust path or a venting path connecting the inside of the container L1 and the outside of the container L1 may be temporarily formed. Also, the container L1 may include a sealing member for re-sealing the inside of the container L1 as the pressure adjusting unit 110 moves out of the container L1 again.

The pressure adjusting unit 110 may exhaust the atmosphere inside the sealed unit 100 or inject the atmosphere outside the sealed unit 100 and may have a needle-like shape for adjusting the internal pressure of the container L1. Also, the pressure adjusting unit 110 may include electrodes constituting the electrode unit 120. In other words, the pressure adjusting unit 110 may include a member having an electrode for forming an electric field inside the sealed unit 100 and exposed into the inside of the sealed unit 100 or a member having an exhaust hole or a venting hole and formed in contact with the outside of the sealed unit 100.

The electrode unit 120 forms an electric field for a process time in which the internal pressure of the sealed unit 100 is within a pre-set process pressure range by using the power supply unit 121, which is an alternated current (AC) power supply, thereby exhausting the low-pressure atmosphere inside the sealed unit 100.

The power supply unit 121 is an AC power supply for alternating voltage at a specific frequency. In this case, the frequency of the power supply unit 121 may be 10 kHz or higher and 200 kHz or lower. This frequency range is a low frequency range and is economically efficient as compared to the conventional plasma treatment apparatus that requires a high frequency AC power of several MHz. Also, the voltage may be applied in a pre-set input waveform, e.g., a sine wave, a triangle wave, a square wave, a sawtooth wave, a pulse wave, etc.

The power supply unit 121 applies a voltage to the first electrode 122 and the second electrode 123 disposed to be spaced apart from the first electrode 122, thereby forming an electric field inside the sealed unit 100. The second electrode 123 is grounded to form an electric field by a voltage difference between the first electrode 122 and the second electrode 123.

The first electrode 122 may be disposed to be spaced apart from the second electrode 123 by a non-conductive member. Also, the first electrode 122 may be disposed to face the second electrode 123. For example, the sealed unit 100 may include a non-conductive member, and the first electrode 122 or the second electrode 123 may be disposed to be spaced apart from the sealed unit 100.

Also, the electrode unit 120 may further include a third electrode disposed to be spaced apart from the first electrode 121 and the second electrode 122. The third electrode may be applied with a voltage of a level same as or different from that of a voltage applied to the first electrode or the second electrode or a voltage and may be a floating electrode that is not electrically connected.

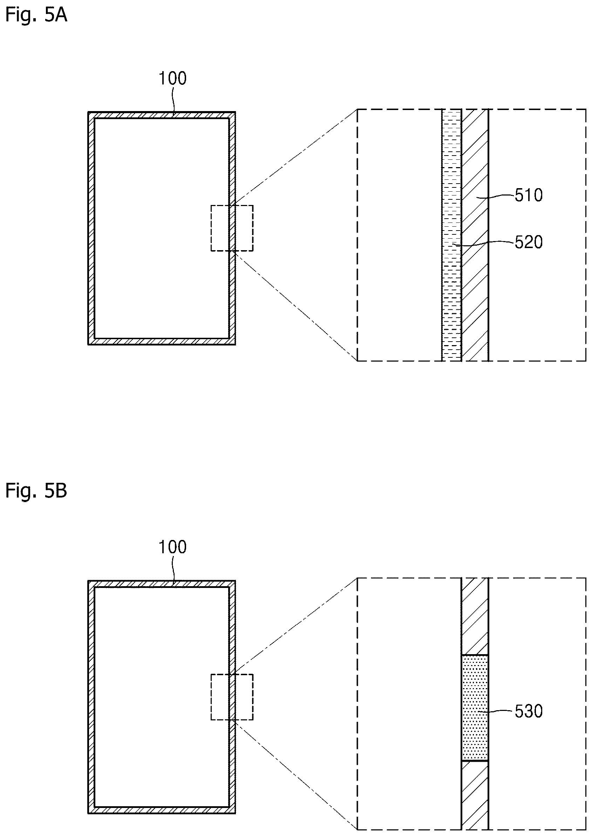

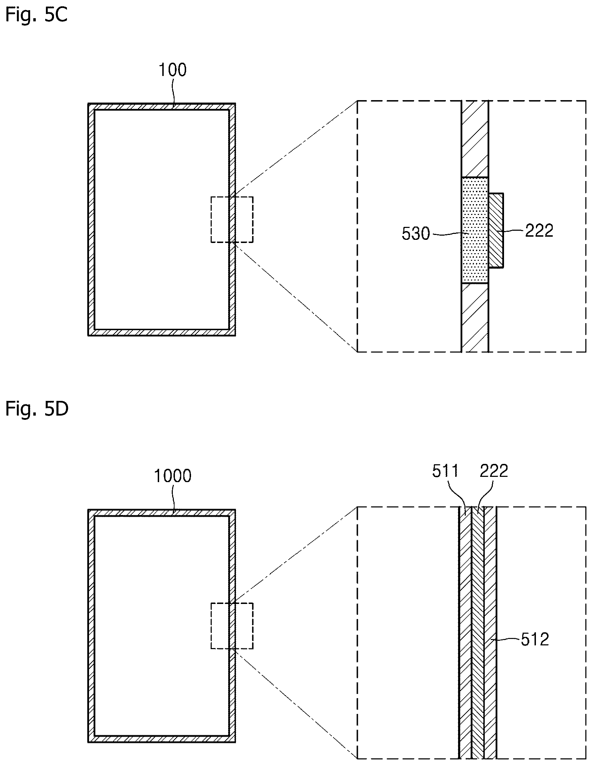

FIGS. 5A to 5D are diagrams for describing other embodiments of the sealed unit of the plasma treatment apparatus of FIG. 4.

Referring to FIG. 5A, in the sealed unit 100, a chemical resistant coating layer 520 is formed inside a base member 510. Alternatively, the base member 510 may include a material having chemical resistance. The sealed unit 100 having an inner surface with chemical resistance prevents contamination due to impurities eluted by plasma generated therein and ionized on the object to be treated. Furthermore, the inner surface with chemical resistance prevents damage that lowers transparency as described later, thereby providing user reliability for the operation of the plasma treatment apparatus.

The chemical resistant coating layer 520 may include a bioactive material including calcium. As such, when the chemical resistant coating layer 520 includes a bioactive material, the elution of calcium is induced by a plasma treatment, and thus calcium may adhere to the surface of the object M1 to be treated. As such, by artificially adhering calcium to the surface of the object M1 to be treated, when implanting or engrafting the object M1 to be treated in the human body, the inflammatory reaction may be relieved or the implantation or engraftment may be strengthened, thereby guaranteeing high implantation rate or high engraftment rate.

Referring to FIGS. 5B to 5D, the sealed unit 100 includes a member 530 including at least a portion made of a transparent material, and thus the low-pressure atmosphere generated inside the sealed unit 100 may be visually confirmed from the outside. Therefore, the plasma treatment apparatus according to the disclosure is configured, such that a surface treatment process may be visually checked from the outside, and thus a user may check the operation of the plasma treatment apparatus simply and intuitively. As a result, the plasma treatment apparatus may have improved reliability.

The transparent material may be a glass material.

At this time, electrodes 222 may be arranged on the inner peripheral surface or the outer peripheral surface of the transparent portion of the member 530 of the sealed unit. At this time, to prevent deterioration of transparency due to the electrodes 222, the electrodes 222 may be formed as transparent electrodes or may have a shape having a specific pattern for ensuring visibility of the inside of the sealed unit, such as a mesh-like shape.

Also, the sealed unit 100 may have a double-walled structure in which the electrodes 222 including a transparent material are arranged. The electrodes 222 may be arranged between an inner wall 511 and an outer wall 512. Here, both the inner wall 511 and the outer wall 512 may have transparent portions that coincide in a viewing direction of a user. Also, the inner wall 511 may be a chemical resistant coating layer or a glass material layer.

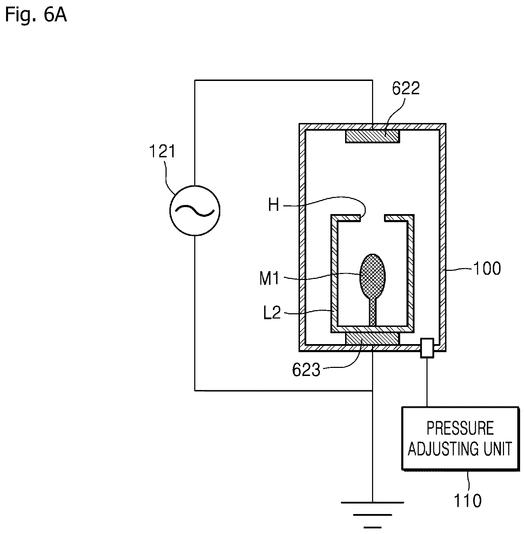

FIGS. 6A to 6D are diagrams for describing other embodiments of the electrode unit of the plasma treatment apparatus of FIG. 4.

Referring to FIG. 6A, the electrode unit 120 includes a first electrode 622 or a second electrode 623 at least a partially exposed into the inside of the sealed unit 100. Also, the first electrode 622 may cause a dielectric barrier discharge based on a voltage difference between the first electrode 622 and the second electrode 623 due to the non-conductive sealed unit 100 or a container having accommodated or stored therein the object to be treated. In other words, at least a portion of the sealed unit 100 or the container becomes a dielectric barrier layer.

At this time, the first electrode 622 and/or the second electrode 623 at least partially exposed into the inside of the sealed unit 100 includes a portion having a sealed structure, the portion connected to the sealed unit 100 to form a vacuum boundary at which the internal sealing of the sealed unit 100 is maintained. In other words, the first electrode 622 and/or the second electrode 623 at least partially exposed into the inside of the sealed unit 100 forms a sealed region with the sealed unit 100. The first electrode 622 or the second electrode 623 includes a hole through which internal air is exhausted or external air is injected to adjust the internal pressure of the sealed unit 100, thereby forming a flow path interconnecting the pressure adjusting unit 110 and the sealed unit 100.

Also, the first electrode 622 and the second electrode 623 may be arranged to face the object M1 to be treated or a container L2 having accommodated therein the object M1 to be treated in the longitudinal direction.

The container L2 having accommodated or stored therein the object M1 to be treated may have a hole H. Although it is exemplified in the drawing that the hole H is formed in the upper portion of the container L2, the position, the shape, and the number of the hole H are not limited. Also, the hole H of the container L2 may be formed in a direction opposite to the first electrode 622 or the second electrode 623. The container L2 may have an internal pressure balanced with that of the inside of the sealed unit 100 through the hole H and changes the strength of an electric field to induced plasma into the container L2. When the container L2 is completely sealed, since there is a large restriction in forming a plasma discharge around the object M1 to be treated accommodated in the container L2, the hole H enables stable plasma generation around the object M1 to be treated with simpler configuration.

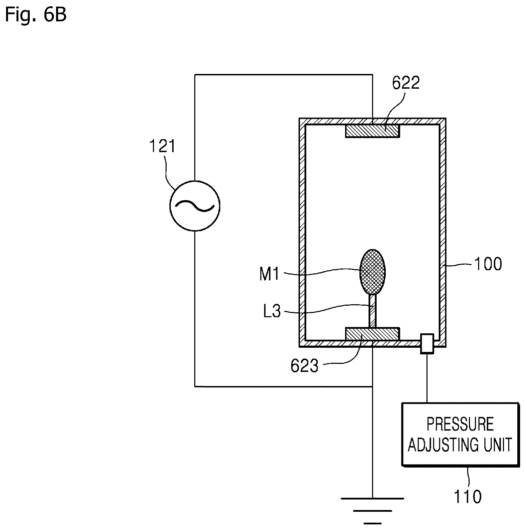

Referring to FIG. 6B, a portion of the second electrode 623 exposed into the inside of the sealed unit 100 is electrically connected to the object M1 to be treated through a conductive member L3. Through the electrical connection, the shape and the intensity of plasma generated around the object M1 to be treated may be controlled, thereby enabling stable generation of plasma with higher efficiency.

The conductive member L3 is provided in the container L2 described with reference to FIG. 6A, has one surface exposed to the outside of the container L2, and is electrically connected to the second electrode 623 of the plasma treatment apparatus. Although the drawing exemplifies that the second electrode 623 is a grounded electrode and the object M1 to be treated faces the first electrode 622, which is an electrode having applied thereto a voltage, the second electrode 623 may be an electrode having applied thereto a voltage and the first electrode 622 may be a ground electrode. Alternatively, the second electrode 623 may be directly electrically connected to a portion of the object M1 to be treated. At this time, the second electrode 623 may have a shape for entering into the container L2 to be electrically connected directly to the object M1 to be treated or to a conductive member contacting the object M1 to be treated, while the object M1 to be treated is being accommodated in the container L2.

Also, the first electrode 622 may become closer to the object M1 to be treated than to the second electrode 623, and thus the shape or the intensity of an electric field may be concentrated around the object M1 to be treated. At this time, the second electrode 623 may use a surface that is in electrical contact with the object M1 to be treated or the container L2 to reduce a surface that is not exposed or exposed in the sealed unit 100, thereby inducing more intensive plasma discharge on the object M1 to be treated.

The conductive member L3 may not be electrically connected to the object M1 to be treated and may be configured as a part of the container L2 to concentrate plasma generation around the object M1 to be treated. For example, the conductive member L3 may be exposed in a region in which the non-conductive object M1 to be treated of the container L2 is accommodated or may be formed as a thin non-conductive member buried in the region.

In other words, the second electrode 623 is electrically connected to an object to be treated accommodated in the sealed unit 100 or a portion of a container having accommodated therein the object to be treated through the portion of the second electrode 623 exposed into the inside of the sealed unit 100.

At this time, the second electrode 623 has a magnetic property to stably contact the conductive member L3 to ensure stability of electrical connection. To this end, a magnet may be configured to be in contact with the second electrode 623.

Also, the conductive member L3 may be a jig that is coupled to the object M1 to be treated drawn out of the container.

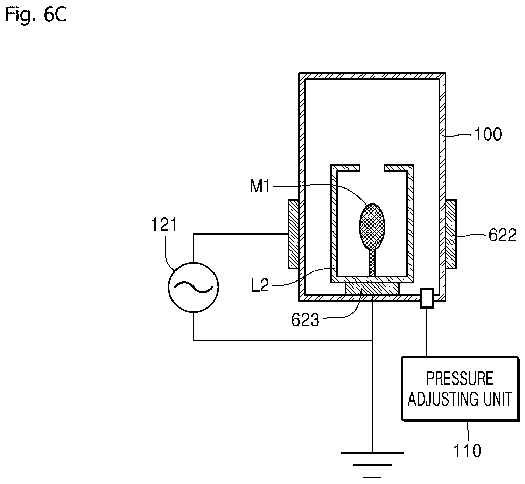

Referring to FIG. 6C, the second electrode 623 constituting the electrode unit 120 may be disposed on the inner peripheral surface or the outer peripheral surface of the sealed unit 100. At this time, the sealed unit 100 includes an accommodating hole corresponding to the shape of the object M1 to be treated or the container L2, and the second electrode 623 is disposed on the inner circumferential surface or the outer circumferential surface of the accommodating hole. Therefore, an electric field is formed adjacent to the object M1 to be treated, and plasma generation is concentrated around the object M1 to be treated. Referring to FIG. 4, the lower member 102 includes an accommodating hole corresponding to the shape of an object to be treated or a container having accommodated therein the object to be treated, and the first electrode 622 and/or the second electrode 623 constituting the electrode unit 120 are arranged on the inner circumferential surface or the outer circumferential surface of the accommodating hole.

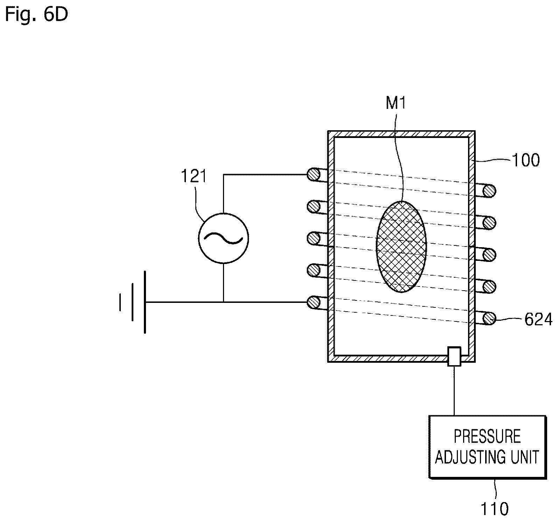

Referring to FIG. 6D, an electrode 624 constituting the electrode unit 120 generates plasma using an inductively coupled plasma (ICP) method. The coil-shaped electrode 624 surrounds the sealed unit 100 and, as power is applied through an AC power supply 221, forms an induced electric field inside the sealed unit 100. Plasma generation method is not limited to those shown in the drawings, like the dielectric barrier discharge (DBD) as shown in FIGS. 6a to 6c, and methods like capacitively coupled plasma (CCP), plasma jet, capillary discharge, micro-discharge, electron cyclotron resonance (ECR), surface wave plasma (SWP), helicon plasma, e-beam plasma, pulsed DC discharge, dual frequency plasma, and hyper-thermal neutral beam plasma may be applied.

Through electrodes arranged adjacent to the object to be treated or in correspondence to the shape of the object to be treated, the plasma treatment apparatus according to embodiments of the disclosure may exhibit high surface treatment efficiency through plasma generation by the atmosphere at a low pressure due to intensive discharged around the object to be treated.

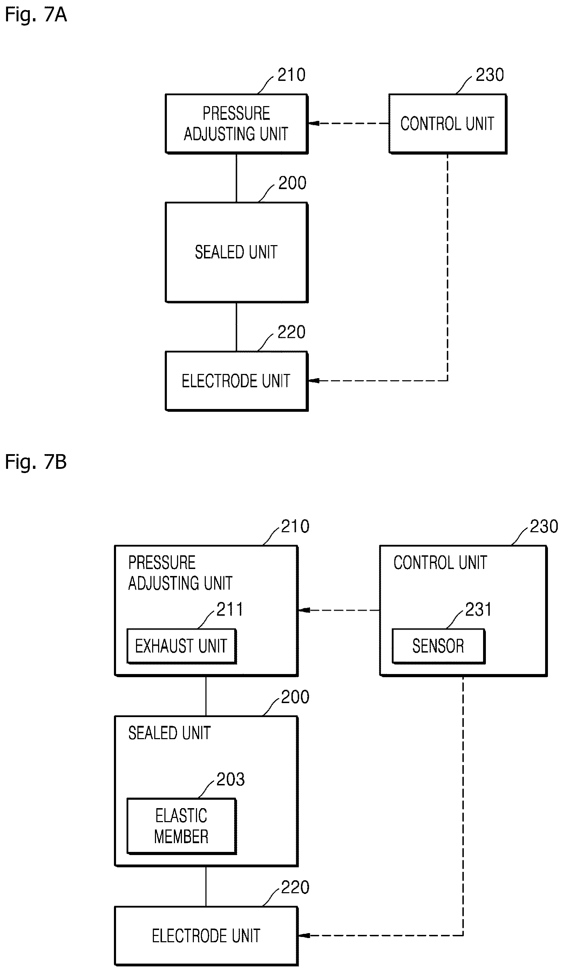

FIGS. 7A to 7F are block diagrams showing a plasma treatment apparatus according to an embodiment of the disclosure. As shown in FIGS. 7A to 7F, the plasma treatment apparatus includes a sealed unit 200, a pressure adjusting unit 210, an electrode unit 220, and a control unit 230. Here, the sealed unit 200, the pressure adjusting unit 210, and the electrode unit 220 may correspond to the sealed unit 100, the pressure adjusting unit 110, and the electrode unit 120 described above, respectively.

Referring to FIG. 7A, the control unit 230 controls the pressure adjusting unit 210 to exhaust the internal atmosphere of the sealed unit 200 to form the low pressure atmosphere within a pre-set process pressure range inside the sealed unit 200 and controls the electrode unit 220, such that discharge occurs in the low-pressure atmosphere inside the sealed unit 200.

Referring to FIG. 7B, the sealed unit 200 may be configured to include an elastic member 203, the pressure adjusting unit 210 may be configured to include an exhaust unit 211, and the control unit 230 may be configured to include a sensor 231.

The elastic member 203 may correspond to the above-stated elastic member 103.

The exhaust unit 211 exhausts the inside of the sealed unit 200.

The control unit 230 controls the exhaust unit 211 to adjust the internal pressure of the sealed unit 200, such that the inside of the sealed unit 200 is sealed against the external environment.

The elastic member 203 is deformed, such that the inside of the sealed unit 200 is sealed against the external environment by using the pressure difference between the inside and the outside of the sealed unit 200. The elastic member 203 is deformed by receiving an inward force from the outside of the sealed unit 200 due into the sealed unit 200 having a lower pressure as compared to the outside of the sealed unit 200, thereby improving the sealing of the inside the sealed unit 200.

The sensor 231 measures a difference between a pressure of the inside and a pressure of the outside of the sealed unit 200.

To measure the internal pressure of the sealed unit 200, the sensor 231 may be disposed inside the sealed unit 200. Alternatively, the sensor 231 may be disposed on a flow path through which the exhaust unit 211 and the sealed unit 200 communicate with each other and measure the pressure of the flow path, thereby measuring the internal pressure of the sealed unit 200. Alternatively, the sensor 231 may measure the internal pressure of the sealed unit 200 by measuring a flow rate flowing into or out of the flow path through which the exhaust unit 211 and the sealed unit 200 communicate with each other.

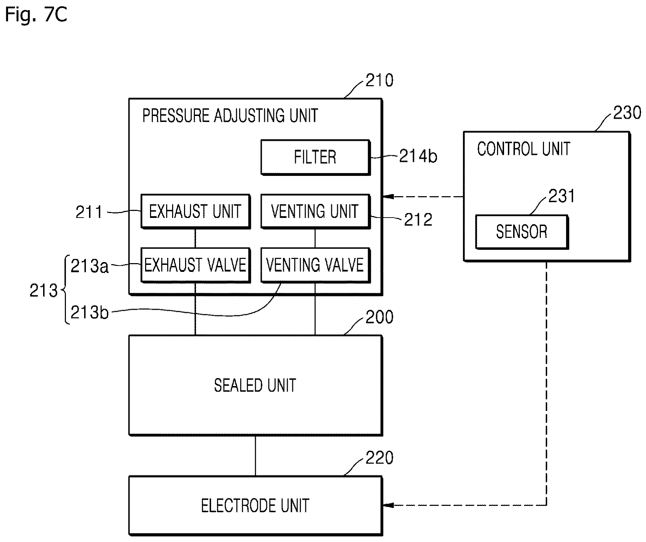

Referring to FIG. 7C, the pressure adjusting unit 210 includes the exhaust unit 211, a venting unit 212, a valve 213, and a filter 214b, and the control unit 230 includes the sensor 231.

The control unit 230 controls the pressure adjusting unit 210 to constantly maintain the internal pressure of the sealed unit 200 within a pre-set process pressure range, such that discharge occurs inside the sealed unit 200 in which the low-pressure atmosphere is constantly maintained.

The pressure adjusting unit 210 includes a vacuum pump that exhausts the internal atmosphere of the sealed unit 200, and the control unit 230 continuously operates the vacuum pump to constantly maintain the internal pressure of the sealed unit 200. When a plasma surface treatment is performed while the inside of the sealed unit 200 is being continuously exhausted, impurities desorbed from the surface of an object to be treated are removed from the inside of the sealed unit 200, and thus re-deposition of the impurities on the object to be treated may be prevented. Also, the vacuum pump may be a pump having a maximum degree of vacuum that satisfies the pre-set process pressure range. For example, the maximum vacuum degree of the vacuum pump may be 0.001 Torr or higher and lower than 100 Torr.

The pressure adjusting unit 210 includes an exhaust valve 213a that opens and closes a flow path interconnecting the sealed unit 200 and the vacuum pump, and the control unit 230 maintains the internal pressure of the sealed unit 200 constant by opening and closing the exhaust valve 213a.

For example, the control unit 230 controls the exhaust unit 211 to continue an exhaust operation, and, to maintain the internal pressure of the sealed unit 200 constant, the control unit 230 closes the exhaust valve 213a when the internal pressure of the sealed unit 200 decreases and opens the exhaust valve 213a when the internal pressure of the sealed unit 200 increases. Therefore, the internal pressure of the sealed unit 200 may be maintained constant. Here, a pressure maintained constant does not mean that the pressure is maintained to exactly the same pressure value, but is maintained within a range set to be tolerable during a process operation.

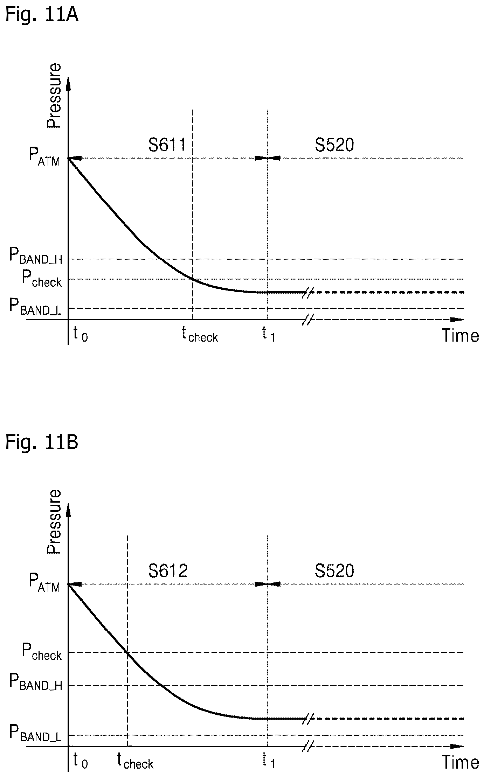

The pressure to be maintained constant may be set in relation to the maximum degree of vacuum of the vacuum pump or may be a reference pressure (P_{check} to be described later, FIGS. 11A to 11C) for checking the internal sealing of the sealed unit 200.

The pressure adjusting unit 210 includes the venting unit 212 for injecting external atmosphere into the sealed unit 200 and a venting valve 213b for opening and closing a flow path interconnecting the venting unit 212 and the sealed unit 200, and the control unit 230 maintains the internal pressure of the sealed unit 200 constant by opening and closing the exhaust valve 213a or the venting valve 213b.

For example, the control unit 230 may close the exhaust valve 213a or open the venting valve 213b when the internal pressure of the sealed unit 200 decreases and may open the exhaust valve 213a or close the venting valve 213b when the internal pressure of the sealed unit 200 increases, thereby maintaining the internal pressure of the sealed unit 200 constant. Also, the exhaust valve 213a or the venting valve 213b may be a valve capable of controlling the degree of closing or opening of a flow path. In this case, the control unit 230 controls the degree of closing or opening of a valve, thereby maintaining the internal pressure of the sealed unit 200 constant.

Alternatively, the exhaust unit 211 may be a pump capable of controlling exhaust power. In this case, the control unit 230 controls the exhaust power of the exhaust unit 211, thereby maintaining the internal pressure of the sealed unit 200 constant.

The control unit 230 injects external atmosphere into the sealed unit 200 during a plasma surface treatment process, and thus nitrogen and oxygen in the injected external atmosphere may be supplied into the sealed unit 200 and increase the generation of active species.

At this time, the pressure adjusting unit 210 further includes the filter 214b that filters the external atmosphere injected into the sealed unit 200. The filter 214b functions to filter or purify contaminants included in external atmosphere and may include, for example, a High Efficiency Particulate Air (HEPA) filter. Therefore, the plasma treatment apparatus may prevent secondary contamination of an object to be treated due to injected external atmosphere.

Also, the sensor 231 may be disposed on a flow path through which the venting unit 212 and the sealed unit 200 communicate with each other and measure the pressure of the flow path thereby measuring the internal pressure of the sealed unit 200. Alternatively, the sensor 231 may measure the internal pressure of the sealed unit 200 by measuring a flow rate flowing into or out of the flow path through which the venting unit 212 and the sealed unit 200 communicate with each other.

The control unit 230 controls the pressure adjusting unit 210 to change the internal pressure of the sealed unit 200 within a pre-set process pressure range, such that discharge occurs inside the sealed unit 200 in which the low-pressure atmosphere varies.

The pressure adjusting unit 210 includes the exhaust unit 211 for exhausting the internal atmosphere of the sealed unit 200 and the exhaust valve 213a for opening and closing a flow path interconnecting the sealed unit 200 and the exhaust unit 211, and the control unit 230 changes the internal pressure of the sealed unit 200 by opening and closing the exhaust valve 213a.

The pressure adjusting unit 210 includes the venting unit 212 for injecting external atmosphere into the sealed unit 200 and a venting valve 213b for opening and closing a flow path interconnecting the venting unit 212 and the sealed unit 200, and the control unit 230 changes the internal pressure of the sealed unit 200 by opening and closing the exhaust valve 213a and/or the venting valve 213b.

The control unit 230 controls the pressure adjusting unit 210 to repeatedly increase and decrease the internal pressure of the sealed unit 200, such that discharge occurs in the low-pressure atmosphere inside the sealed unit 200 whose pressure is repeatedly changed.

The plasma treatment apparatus according to an embodiment of the disclosure controls plasma generation due to low-pressure atmosphere inside the sealed unit 200 through the pressure control as described above, thereby improving efficiency of surface treatment including removal of impurities on surfaces of an object to be treated accommodated in the plasma treatment apparatus.

The plasma treatment apparatus according to an embodiment of the disclosure may perform a surface treatment process by combining a first surface treatment process for discharging while maintaining the internal pressure of the sealed unit 200 constant and a second surface treatment process for discharging while changing the internal pressure of the sealed unit 200. Here, in the first surface treatment process, the process pressure range may be pre-set within the range of 2 Torr or higher and less than 30 Torr, and, in the second surface treatment process, the process pressure range may be pre-set within the range of 1 Torr or higher and less than 100 Torr. In other words, the first surface treatment process may be configured to perform surface treatment through plasma discharges with repetition reliability in constant shape and intensity by setting a narrower process pressure range, and the second surface treatment process may be configured to perform surface treatment with improved efficiency by changing shape, intensity, or concentration of plasma by setting a wider process pressure range.

The control unit 230 may control the pressure adjusting unit 210 or the electrode unit 220 based on the internal pressure of the sealed unit 200 measured through the sensor 231 or based on a pre-set time. For example, the control unit 230 controls according to a pre-set protocol. The control unit 230 controls the pressure adjusting unit 210 or the electrode unit 220 based on a pre-set time based on a time related to at least one of exhaust, venting, power application, and accommodation of an object to be treated.

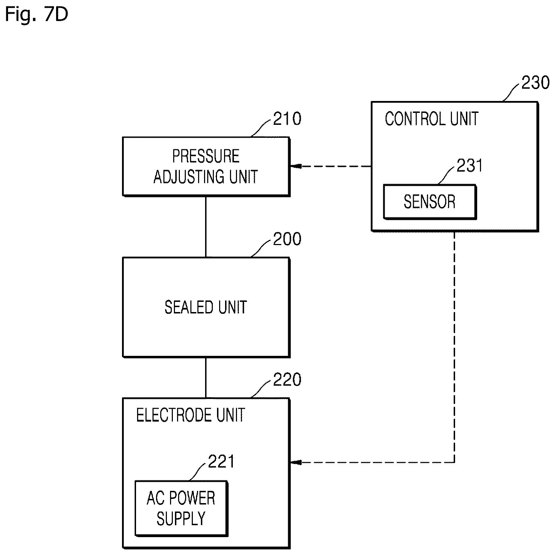

Referring to FIG. 7D, the electrode unit 220 includes an AC power supply 221, and the control unit 230 includes the sensor 231.

The AC power supply 221 forms an electric field inside the sealed unit 200 for a process time in which the internal pressure of the sealed unit 200 is within a pre-set process pressure range.

The control unit 230 controls to keep the AC power supply 221 ON during the process time.

Alternatively, the control unit 230 repeatedly turns the AC power supply 221 ON and OFF during the process time. Therefore, the shape and the intensity of an electric field formed inside the sealed unit 200 are changed, and thus, as plasma is generated and extinguished, the shape, the intensity, and the concentration of plasma are changed. When only plasma generation is continued, the plasma discharge state is stabilized and the surface treatment effect decreases over time. When plasma is generated and extinguished repeatedly in the form of pulses, it is possible to prevent a deterioration of efficiency due to stabilization of the plasma discharge state. Also, damage to an object to be treated due to plasma may be reduced, and the process temperature may be lowered.

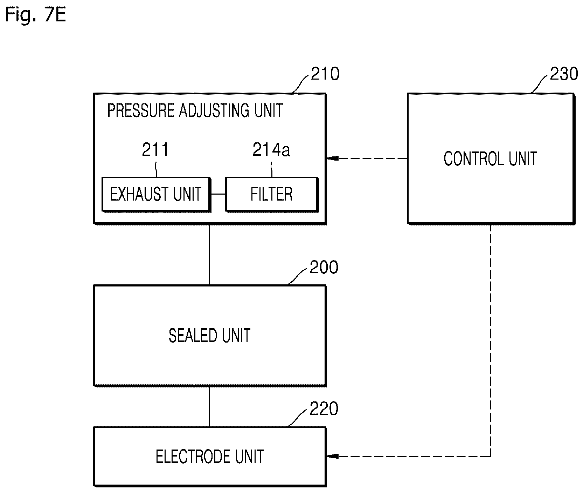

Referring to FIG. 7E, the pressure adjusting unit 210 includes the exhaust unit 211 and a purification filter 214a.

The control unit 230 controls the exhaust unit 211 to remove by-products generated due to the discharge of the low-pressure atmosphere inside the sealed unit 200 from the inside of the sealed unit 200.

The purification filter 214a purifies the by-products removed from the inside of the sealed unit 200 through the exhaust unit 211. For example, the purification filter 214a may be an ozone (O₃) filter. Also, the purification filter 214a is disposed in a flow path for exhausting through the exhaust unit 211, and thus by-products are exposed to the outside of the plasma treatment apparatus through the purification filter 214a. Therefore, the plasma treatment apparatus may maximize the effect of enhancing user safety by controlling the external discharge of by-products generated by discharging of the low-pressure atmosphere.

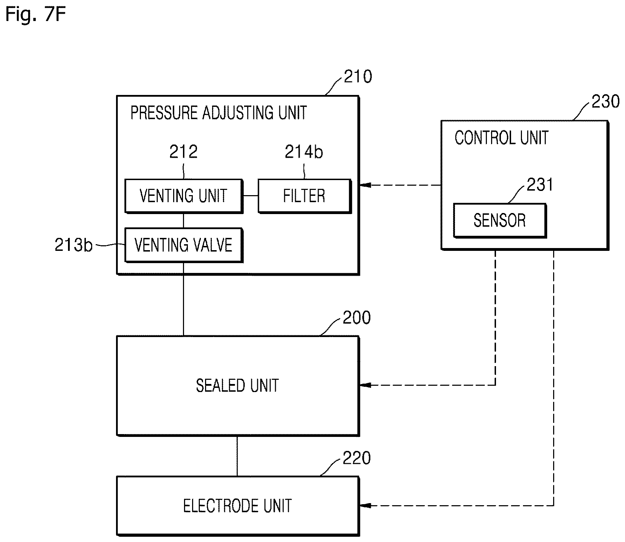

Referring to FIG. 7F, the pressure adjusting unit 210 includes the venting unit 212, the venting valve 213b, and the filter 214b, and the control unit 230 includes the sensor 231.

The venting unit 212 has an open region exposed to the external atmosphere and injects the external atmosphere into the sealed unit 200. The venting valve 213b opens and closes a flow path interconnecting the sealed unit 200 and the venting unit 212. When plasma treatment is completed, the control unit 230 opens the venting valve 213b to form an atmospheric flow according to a pressure difference between the internal pressure of the sealed unit 200 and the pressure of the external atmosphere.

The control unit 230 controls the opening of the sealed unit 200 after adjusting the internal pressure of the sealed unit 200 to be equalized with the pressure of the external atmosphere by using the atmospheric flow.

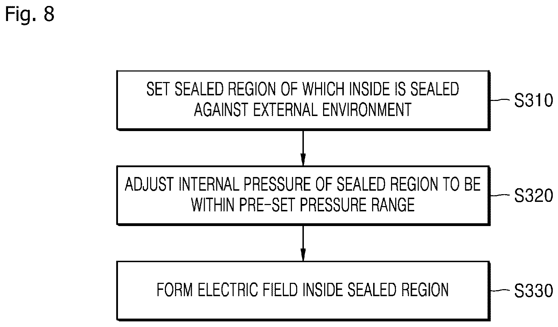

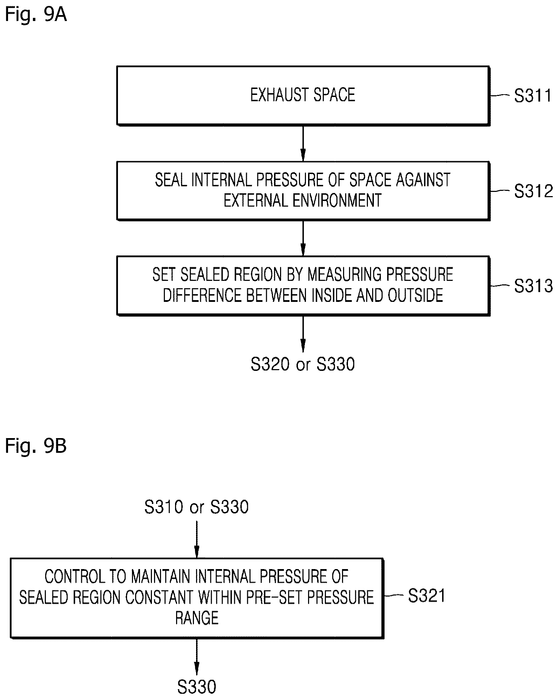

FIG. 8 is a flowchart for describing a process operation of the plasma treatment apparatus of FIG. 1, and FIGS. 9A to 9E are flowcharts for describing another embodiment of FIG. 8.

Referring to FIG. 8, the plasma treatment apparatus of the disclosure performs a process operation including operation S310 of setting a sealed region of which the inside is sealed against the external environment, operation S320 of adjusting the internal pressure of the sealed region within a pre-set process pressure range, and operation S330 of forming an electric field inside the sealed region.

Operation S310 may further include placing an object to be treated in a sealed region of which the inside is sealed against the external environment before the sealed region is set.

Referring to FIG. 9A, operation S310 may include operation S311 of exhausting a space, operation S312 of sealing the internal pressure of the space against the external environment, and operation S313 of setting a sealed region by measuring a pressure difference between the inside and the outside of the space.

Also, operation S313 may include operation S320, and operation S330 may be performed after operation S313.

Referring to FIG. 9B, the plasma treatment apparatus of the disclosure performs a process operation including operation S321 of controlling the internal pressure of the sealed region to be constantly maintained within a pre-set process pressure range after operation S310 or S330.

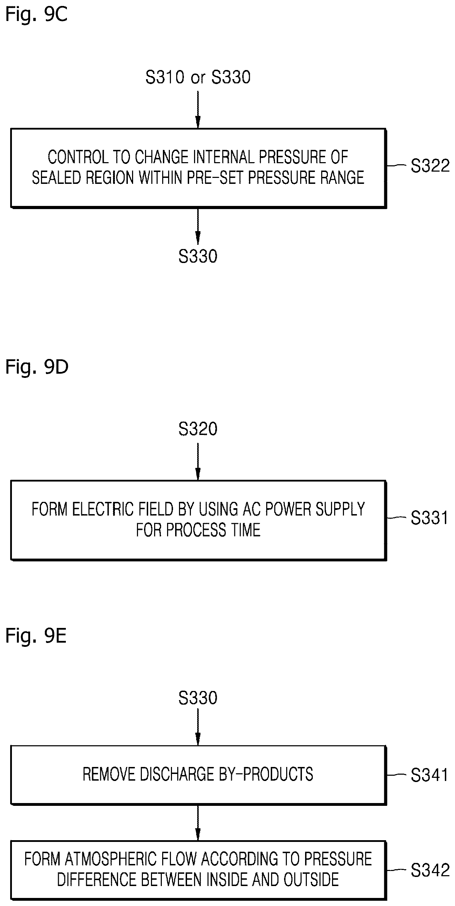

Referring to FIG. 9C, the plasma treatment apparatus of the disclosure performs a process operation including operation S322 of controlling the internal pressure of the sealed region to vary within the pre-set process pressure range after operation S310 or S330.

Referring to FIG. 9D, the plasma treatment apparatus of the disclosure performs a process operation including operation S331 of forming an electric field by using an AC power supply during a process time after operation S310 or S320.

Referring to FIG. 9E, the plasma treatment apparatus of the disclosure further performs operation S341 of removing discharge by-products after operation S330 and operation S342 of forming an atmospheric flow according to a pressure difference between the inside and the outside of the sealed region.

Operation S342 may further include releasing the sealing of the sealed region after the pressure inside the sealed region and the pressure outside the sealed region are equalized. Also, operation S342 may further include opening the sealed unit having an internal pressure balanced with the outside and discharging an object to be treated, which is accommodated in the sealed region and is surface-treated.

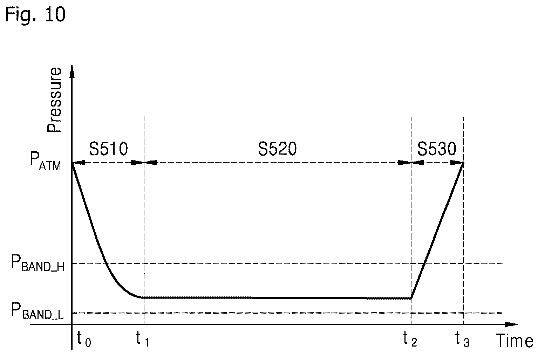

FIG. 10 is a graph showing changes in the internal pressure of the sealed unit of the plasma treatment apparatus of FIG. 1 over time.

FIG. 10 shows changes divided into a section S510 in which the internal pressure of the sealed unit 100 starts to decrease to seal the inside of the sealed unit 100 against the external environment during a plasma treatment process, a section S520 in which the internal pressure is in a pre-set process pressure range, and a section S530 in which the internal pressure starts to increase to be equalized to the pressure outside the sealed unit 100. The electrode unit 120 may form an electric field inside the sealed unit 100 in the section S520.

As shown in FIG. 10, in the section S520, the internal pressure of the sealed unit 100 may be constantly maintained within the pre-set process pressure range.

FIGS. 11A to 11I are graphs showing other embodiments in which the internal pressure of the sealed unit of FIG. 10 changes over time.

As shown in FIGS. 11A to 11C, in the section S510 in which the internal pressure of the sealed unit 100 starts to decrease to seal the inside of the sealed unit 100 against the external environment, it is checked whether the inside of the sealed unit 100 is sealed.

As shown in FIG. 11A, the plasma treatment apparatus checks whether the internal pressure of the sealed unit 100 has reached a reference pressure P_{check} included in a pre-set process pressure range. Alternatively, the plasma treatment apparatus measures the internal pressure of the sealed unit 100 in a pre-set time t_{check} and evaluates whether the internal pressure is lower than the reference pressure P_{check}. Alternatively, the plasma treatment apparatus checks whether the inside of the sealed unit 100 is sealed by measuring an amount of change in the internal pressure of the sealed unit 100 during a set time t_{check} or a set section.

As shown in FIG. 11B, the plasma treatment apparatus checks whether the internal pressure of the sealed unit 100 has reached the reference pressure P_{check} exceeding the pre-set process pressure range. Alternatively, the plasma treatment apparatus measures the internal pressure of the sealed unit 100 in the pre-set time t_{check} and evaluates whether the internal pressure is lower than the reference pressure P_{check}.

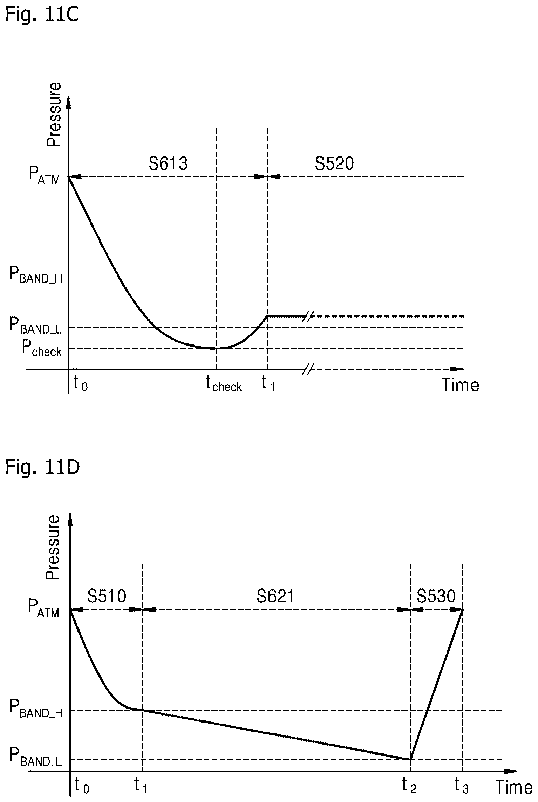

As shown in FIG. 11C, the plasma treatment apparatus checks whether the internal pressure of the sealed unit 100 has reached the reference pressure P_{check} lower than the pre-set process pressure range.

The reference pressure P_{check} or the time t_{check} in FIGS. 11A to 11C may be pre-set in relation to the maximum vacuum degree or pumping efficiency of the vacuum pump constituting the pressure adjusting unit 110.

In FIGS. 11A to 11C, the plasma treatment apparatus may check whether the the inside of the sealed unit 100 is sealed, determine that there is an error related to the sealing of the sealed unit 100 or an operation error of the pressure adjusting unit 110, stop the operation of the plasma treatment apparatus, and notify a user the error or the operation error.

As shown in FIGS. 11D to 11H, the internal pressure of the sealed unit 100 may vary within a pre-set process pressure range after the section S510 in which the internal pressure reaches the pre-set process pressure range.

As shown in FIG. 11D, there is a section S621 in which the internal pressure of the sealed unit 100 decreases within the pre-set process pressure range. The phenomenon may appear when the pressure adjusting unit 110 continuously exhausts the inside of the sealed unit 100 or performs controlled exhaust.

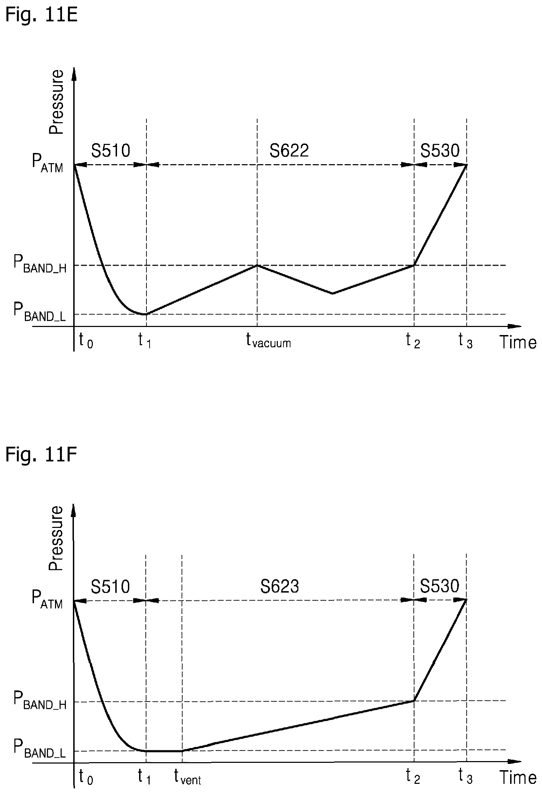

As shown in FIG. 11E, there is a section S622 in which the internal pressure of the sealed unit 200 is changed according to being controlled within the pre-set process pressure range. The phenomenon may appear as the inside of the sealed unit 100 is exhausted at a time point t_{vacuum} at which the internal pressure of the sealed unit 100 reaches the upper limit of the pre-set process pressure range. In this case, the internal pressure of the sealed unit 200 decreases and increases irregularly within the pre-set process pressure range.

As shown in FIG. 11F, there is a section S623 in which the internal pressure of the sealed unit 200 increases within the pre-set process pressure range. The phenomenon may appear when the pressure adjusting unit 110 constantly injects the external atmosphere into the the sealed unit 100 or performs controlled injection and exhaust. For example, the pressure adjusting unit 110 may inject the external atmosphere into the interior of the sealed unit 100 during a pre-set time tᵥₑₙₜ to provide a section in which the internal pressure of the sealed unit 100 is increased.

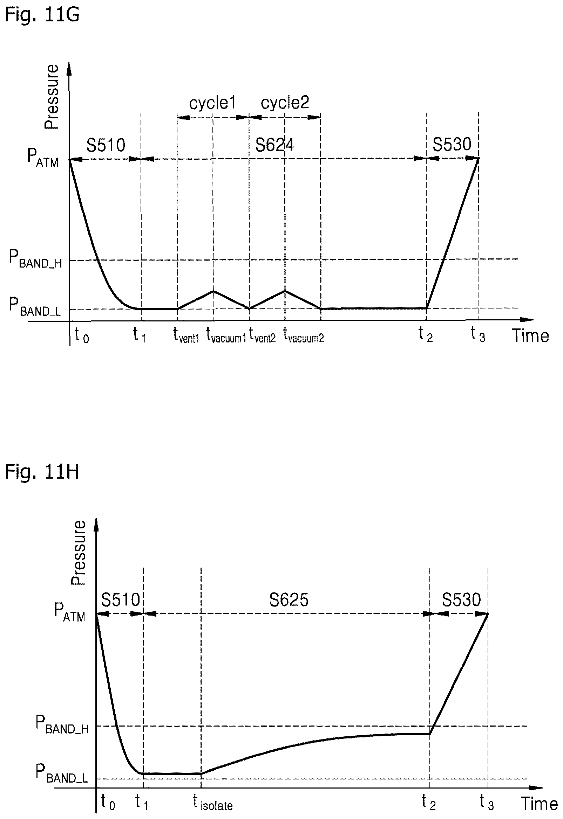

As shown in FIG. 11G, there is a section S624 in which the internal pressure of the sealed unit 100 repeatedly decreases and increases at certain cycles cycle1 and cycle2 within the pre-set process pressure range. The phenomenon may appear when the pressure adjusting unit 110 injects the external atmosphere at certain time intervals tᵥₑₙₜ₁ and tᵥₑₙₜ₂ into the sealed unit 100 or exhaust the inside of the sealed unit 100 at certain time intervals t_{vacuum1} and t_{vacuum2}. A plasma treatment process in which constantly repeated pressure changes inside the sealed unit 100 may provide a surface treatment effect with improved repetition reliability.

As shown in FIG. 11H, there is a section S625 in which the inside of the sealed unit 200 is isolated without being exhausted or vented to the outside. However, in the section S625, even when the inside of the sealed unit 200 is completely blocked and closed from the outside, a leak occurs and the internal pressure of the sealed unit 200 slightly increases. When a plasma surface treatment is performed while the inside of the sealed unit 200 is being closed from the outside, ozone (O₃) generated during the plasma surface treatment is not exhausted, and thus the concentration of ozone (O₃) is maintained or increases. Therefore, as the concentration of ozone (O₃) in the sealed space increases, the sterilization of an object to be treated may be effectively performed. The phenomenon may appear as the pressure adjusting unit 110 isolates the inside of the sealed unit 100 for a pre-set time tᵢₛₒₗₐₜₑ.

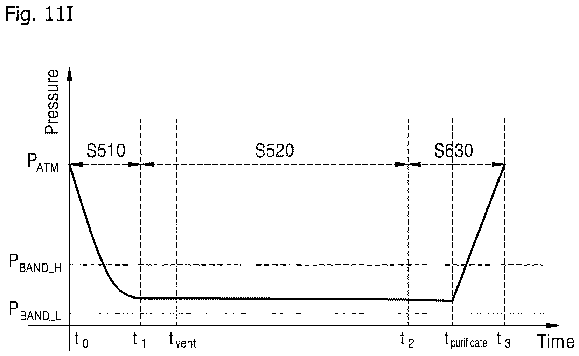

As shown in FIG. 11I, there is a section S630 in which the internal pressure of the sealed unit 100 is within a constantly low pressure range before the internal pressure of the sealed unit 100 increases to be equalized to the pressure outside the sealed unit 100. The phenomenon may appear as the pressure adjusting unit 110 maintains the sealing of the sealed unit 100 against the external environment during a pre-set time t_{purificate} or until a separate input from a user is received. Alternatively, the pressure adjusting unit 110 exhausts the inside of the sealed unit 100 for the pre-set time t_{purificate}, thereby removing by-products from the inside of the sealed unit 100. Thereafter, the pressure adjusting unit 110 makes the internal pressure of the sealed unit 100 to be equalized with the pressure of the external atmosphere.

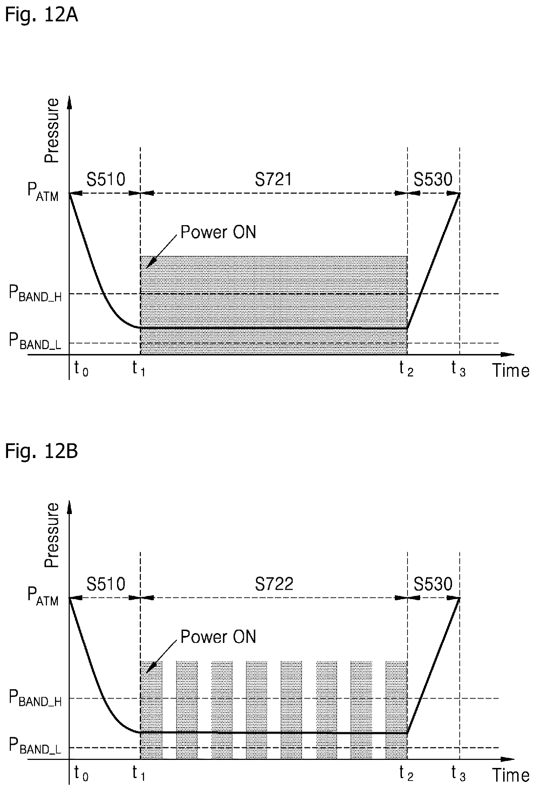

FIGS. 12A to 12C are graphs showing power applied to the electrode unit of the plasma treatment apparatus of FIG. 1.

As shown in FIG. 12A, there is a section S721 in which the electrode unit 120 is maintained ON and an electric field formed inside the sealed unit 100 is continuously maintained after the section S510 in which the internal pressure of the sealed unit 100 reaches the pre-set process pressure range.

As shown in FIG. 12B, there is a section S722 in which formation and extinction of an electric field are repeated inside the sealed unit 100 as the electrode unit 120 is repeatedly turned ON and OFF after the operation S510.

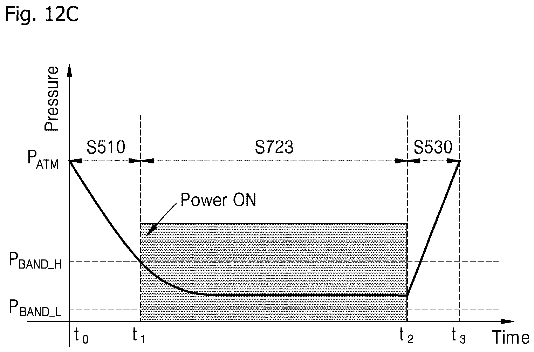

As shown in FIG. 12C, there is a section S723 in which a time point for the electrode unit 120 to start voltage application inside the sealed unit 100 is adjusted after the section S510. Therefore, the plasma treatment apparatus may control the shape, the intensity, and the concentration of plasma related to the plasma surface treatment effect.

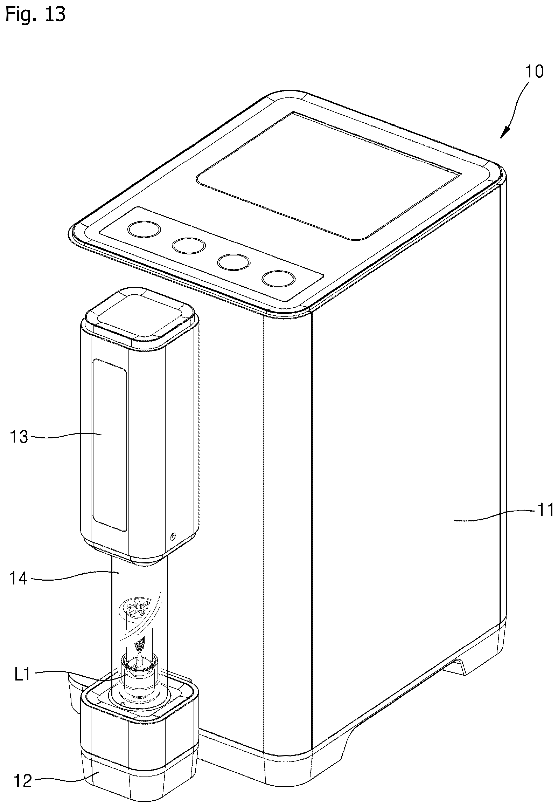

FIG. 13 is a perspective view of a plasma treatment apparatus according to an embodiment of the disclosure.

Referring to FIG. 13, a plasma treatment apparatus 10 may include an accommodating unit 12 on which the container L1 is accommodated, a sealed unit 14 that moves in relation to the accommodating unit 12 to seal the container L1 against the external environment, an electrode unit (not shown) for discharging plasma inside the sealed unit 14 sealed against the external environment, a pressure adjusting unit (not shown) for exhausting the air inside the sealed unit 14 sealed against the external environment, an upper block 13 disposed over the accommodating unit 12, and a main body 11 constituting the exterior of the plasma treatment apparatus 10.

The accommodating unit 12 is disposed to be positioned in front of the main body 11 and may be disposed to be positioned under the upper block 13. An electrode for applying power to the container L1 may be formed on the top surface of the accommodating unit 12.

As shown in the drawing, the container L1 has a hole, and the internal pressure of the container L1 is adjusted together in the process of adjusting the internal pressure of the sealed unit 14, such that the internal pressure of the container L1 is equalized to the internal pressure of the sealed unit 14. In other words, the container L1 has a hole, and the internal atmosphere of the container L1 may be exhausted or the external atmosphere may be injected through the hole.

Also, the strength or the shape of an electric field related to plasma generation is changed through the hole of the container L1, and thus the efficiency of surface treatment for an object to be treated accommodated in the container L1 may be improved.

At this time, a hole (not shown) for accommodating the entire lower portion of the container L1 may be formed in the accommodating unit 12 or, when an electrical connection member (not shown) of the container L1 protrudes from the container L1, a hole (not shown) for accommodating the protruding electrical connection member may be formed in the accommodating unit 12.

Also, a magnet may be provided in the accommodating unit 12 to increase contact force with respect to the electrical connection member (not shown) through magnetic force. The magnet may be provided on the bottom surface of the hole (not shown).

The sealed unit 14 is moved in relation to the accommodating unit 12 and seals the container L1 from the external environment. In the disclosure, according to an embodiment, as the sealed unit 14 is elevated and lowered, the lower portion of the sealed unit 14 contacts the top surface of the accommodating unit 12, and thus a sealed space is formed inside the sealed unit 14.

The upper block 13 may be disposed to be positioned in front of the main body 11 and above the accommodating unit 12. The upper block 13 may be provided with an elevating unit (not shown) for elevating and lowering the sealed unit 14.

The electrode unit (not shown) may perform plasma treatment by discharging plasma into the hollow of the sealed unit 14 constituting a sealed space when the sealed unit 14 is lowered and the accommodating unit 12 and the sealed unit 14 are closed. The electrode unit (not shown) may include a first electrode (not shown) provided in the accommodating unit 12 to be electrically connected to the container L1, a second electrode (not shown) provided in the sealed unit 14 to surround the container L1, and a power supply (not shown) for applying power to the first electrode (not shown) and the second electrode (not shown).

The pressure adjusting unit (not shown) may perform the function of exhausting the air inside the sealed unit 14 sealed against the external environment.

While the present disclosure has been described with reference to exemplary embodiments, it is to be understood that the present disclosure is not limited to the disclosed example embodiments, but, on the contrary, is intended to cover various modifications and equivalent arrangements included within the spirit and scope of the appended claims. Accordingly, the true scope of protection of the present disclosure should be determined by the technical idea of the appended claims.

### Industrial Applicability

According to an embodiment of the disclosure, a plasma treatment apparatus is provided. Also, embodiments of the disclosure may be applied to a technique for sterilizing an object to be treated or treating a surface of an object to be treated by using plasma.
The following description pages 29 to 33 contain preferred embodiments.
Embodiment 1. A plasma treatment apparatus comprising:
   a sealed unit whose inside is sealed against an external environment;
   a pressure adjusting unit configured to adjust an internal pressure of the sealed unit to be within a pre-set process pressure range; and
   an electrode unit configured to form an electric field inside the sealed unit.
Embodiment 2. The plasma treatment apparatus of embodiment 1, further comprising
   a control unit configured to control the pressure adjusting unit to exhaust internal atmosphere of the sealed unit to form low-pressure atmosphere within the pre-set process pressure range inside the sealed unit and
   control the electrode unit to discharge the low-pressure atmosphere.
Embodiment 3. The plasma treatment apparatus of embodiment 2,
   wherein the pre-set process pressure range is set in a range of 1 Torr or higher and less than 100 Torr.
Embodiment 4. The plasma treatment apparatus of embodiment 2,
   wherein the sealed unit comprises an upper member and a lower member that are separated from each other, and
   the upper member or the lower member seals the inside of the sealing unit by moving in relation to each other.
Embodiment 5. The plasma treatment apparatus of embodiment 4,
   wherein the lower member comprises an accommodating hole corresponding to a shape of an object to be treated or a container having accommodated therein the object to be treated, and
   the electrode unit comprises an electrode disposed on an inner circumferential surface of the accommodating hole.
Embodiment 6. The plasma treatment apparatus of embodiment 2,
   wherein the inside of the sealed unit comprises a material having chemical resistance or a chemical resistant coating layer is formed on the inside of the sealed unit.
Embodiment 7. The plasma treatment apparatus of embodiment 2,
   wherein at least a portion of the sealed unit comprises a transparent material, and thus the low-pressure atmosphere in the inside of the sealed unit formed by discharge is visually recognizable from the outside.
Embodiment 8. The plasma treatment apparatus of claim 7,
   wherein the transparent material is a glass material.
9. The plasma treatment apparatus of embodiment 2,
   wherein the electrode unit comprises an electrode, and
   at least a portion of the electrode is exposed into the inside of the sealed unit.
Embodiment 10. The plasma treatment apparatus of embodiment 9,
   wherein the electrode at least partially exposed into the inside of the sealed unit, is electrically connected to an object to be treated accommodated in the sealed unit or a portion of a container having accommodated therein the object to be treated.
Embodiment 11. The plasma treatment apparatus of embodiment 10,
   wherein the electrode is magnetic.
Embodiment 12. The plasma treatment apparatus of embodiment 2,
   wherein the pressure adjusting unit comprises an exhaust unit for exhausting the inside of the sealed unit, and
   the control unit controls the exhaust unit to control the internal pressure of the sealed unit, such that the inside of the sealed unit is sealed against the external environment.
Embodiment 13. The plasma treatment apparatus of embodiment 12,
   wherein the sealed unit comprises an elastic member that is deformed such that the inside of the sealed unit is sealed against the external environment by using a pressure difference between the inside and outside of the sealed unit.
Embodiment 14. The plasma treatment apparatus of embodiment 12,
   wherein the control unit comprises a sensor for measuring a difference between a pressure of the inside and a pressure of the outside of the sealed unit.
Embodiment 15. The plasma treatment apparatus of embodiment 2,
   wherein the control unit controls the pressure adjusting unit to maintain the internal pressure of the sealed unit constant within the pre-set process pressure range, and
   discharges low-pressure atmosphere inside the sealed unit in which the pressure of the low-pressure atmosphere is maintained constant.
Embodiment 16. The plasma treatment apparatus of embodiment 15,
   wherein the pressure adjusting unit comprises a vacuum pump for exhausting the internal atmosphere of the sealed unit, and
   the control unit maintains the internal pressure of the sealed unit constant by continuously operating the vacuum pump and discharges the low-pressure atmosphere inside the sealed unit in which the pressure of the low-pressure atmosphere is maintained constant.
Embodiment 17. The plasma treatment apparatus of embodiment 15,
   wherein the pressure adjusting unit comprises a vacuum pump for exhausting the internal atmosphere of the sealed unit and a valve for opening and closing a flow path interconnecting the sealed unit and the vacuum pump, and
   the control unit maintains the internal pressure of the sealed unit constant by opening and closing the valve and discharges plasma inside the sealed unit in which the pressure of the low-pressure atmosphere is maintained constant.
Embodiment 18. The plasma treatment apparatus of embodiment 15,
   wherein the pressure adjusting unit comprises a vacuum pump for exhausting the internal atmosphere of the sealed unit; a venting unit for injecting external air into the sealed unit; and a valve for opening and closing a flow path interconnecting the vacuum pump or the venting unit and the sealed unit, and
   the control unit maintains the internal pressure of the sealed unit constant by opening and closing the valve and discharges low-pressure atmosphere inside the sealed unit in which the pressure of the low-pressure atmosphere is maintained constant.
Embodiment 19. The plasma treatment apparatus of embodiment 15,
   wherein the pre-set process pressure range is set in a range of 2 Torr or higher and less than 30 Torr.
Embodiment 20. The plasma treatment apparatus of embodiment 2,
   wherein the control unit controls the pressure adjusting unit to change the internal pressure of the sealed unit to be within the pre-set process pressure range, and
   discharges low-pressure atmosphere inside the sealed unit in which the pressure of the low-pressure atmosphere is changed.
Embodiment 21. The plasma treatment apparatus of embodiment 20,
   wherein the pressure adjusting unit comprises an exhaust unit for exhausting the internal atmosphere of the sealed unit and a valve for opening and closing a flow path interconnecting the sealed unit and the exhaust unit, and
   the control unit changes the internal pressure of the sealed unit by opening and closing the valve and discharges low-pressure atmosphere inside the sealed unit in which the pressure of the low-pressure atmosphere is changed.
Embodiment 22. The plasma treatment apparatus of embodiment 20,
   wherein the pressure adjusting unit comprises an exhaust unit for exhausting the internal atmosphere of the sealed unit; a venting unit for injecting external air into the sealed unit; and a valve for opening and closing a flow path interconnecting the exhaust unit or the venting unit and the sealed unit, and
   the control unit changes the internal pressure of the sealed unit by opening and closing the valve and discharges plasma inside the sealed unit in which the pressure of the low-pressure atmosphere is changed.
Embodiment 23. The plasma treatment apparatus of embodiment 2,
   wherein the control unit controls the pressure adjusting unit to repeatedly increase and decrease the internal pressure of the sealed unit,
   thereby discharging the low-pressure atmosphere of which pressure is repeatedly changed inside the sealed unit.
Embodiment 24. The plasma treatment apparatus of any one of embodiments 18, 22 and 23,
   wherein the pressure adjusting unit further comprises a filter for filtering external atmosphere injected into the sealed unit.
Embodiment 25. The plasma treatment apparatus of any one of embodiments 17, 18, and 21 to 23,
   wherein the control unit further comprises a sensor for measuring the internal pressure of the sealed unit.
Embodiment 26. The plasma treatment apparatus of any one of embodiments 17, 18, and 21 to 23,
   wherein the control unit controls the pressure adjusting unit based on a pre-set time.
Embodiment 27. The plasma treatment apparatus of embodiment 2,
   wherein the electrode unit forms the electric field with an alternating current (AC) power supply for a process time during which the internal pressure of the sealed unit is within the pre-set process pressure range and discharges the low-pressure atmosphere.
Embodiment 28. The plasma treatment apparatus of embodiment 27,
   wherein the control unit maintains the AC power supply ON during the process time to discharge the low-pressure atmosphere.
Embodiment 29. The plasma treatment apparatus of embodiment 27,
   wherein the control unit repeatedly turns the AC power supply ON and OFF during the process time to discharge the low-pressure atmosphere.
Embodiment 30. The plasma treatment apparatus of embodiment 27,
   wherein a frequency of the AC power supply is 10 kHz or higher and 200 kHz or lower.
Embodiment 31. The plasma treatment apparatus of embodiment 2,
   wherein the pressure adjusting unit comprises an exhaust unit for exhausting the inside of the sealed unit, and
   the control unit controls the exhaust unit to remove by-products, which are generated by discharging the low-pressure atmosphere inside the sealed unit, from the inside of the sealed unit.
Embodiment 32. The plasma treatment apparatus of embodiment 31,
   wherein the exhaust unit further comprises a filter for purifying the by-products.
Embodiment 33. The plasma treatment apparatus of embodiment 2,
   wherein the pressure adjusting unit comprises a venting unit exposed to external atmosphere and a valve for opening and closing a flow path interconnecting the sealed unit and the venting unit, and
   the control unit opens the valve to form an atmospheric flow according to a pressure difference between the internal pressure of the sealed unit and the pressure of the external atmosphere.
Embodiment 34. The plasma treatment apparatus of embodiment 33,
   wherein the control unit controls opening of the sealed unit by allowing the internal pressure of the sealed unit to be equalized to an external pressure by the atmospheric flow.
Embodiment 35. The plasma treatment apparatus of embodiment 2,
   wherein the object to be treated is accommodated inside the sealed unit and a surface of the object to be treated is treated, and
   the object to be treated is selected from a group consisting of dental implants, orthopedic implants, bone grafts, skin grafts, ophthalmic implants, heart implants, cochlear implants, cosmetic implants, nerve implants, medical resins, dental prostheses, fibers, seeds, and foods.

## Claims

1. A plasma treatment apparatus comprising:
a sealed unit whose inside is sealed against an external environment;
a pressure adjusting unit configured to adjust an internal pressure of the sealed unit to be within a pre-set process pressure range; and
an electrode unit configured to form an electric field inside the sealed unit.

2. The plasma treatment apparatus of claim 1, further comprising
a control unit configured to control the pressure adjusting unit to exhaust internal atmosphere of the sealed unit to form low-pressure atmosphere within the pre-set process pressure range inside the sealed unit and
control the electrode unit to discharge the low-pressure atmosphere.

3. The plasma treatment apparatus of claim 1 or 2,
wherein the pre-set process pressure range is set in a range of 1 Torr or higher and less than 100 Torr.

4. The plasma treatment apparatus of any one of claims 1 to 3,
wherein the sealed unit comprises an upper member and a lower member that are separated from each other, and
the upper member or the lower member seals the inside of the sealing unit by moving in relation to each other.

5. The plasma treatment apparatus of claim 4,
wherein the lower member comprises an accommodating hole corresponding to a shape of an object to be treated or a container having accommodated therein the object to be treated, and
the electrode unit comprises an electrode disposed on an inner circumferential surface of the accommodating hole.

6. The plasma treatment apparatus of claim 2,
wherein at least a portion of the sealed unit comprises a transparent material, and thus the low-pressure atmosphere in the inside of the sealed unit formed by discharge is visually recognizable from the outside.

7. The plasma treatment apparatus of any one of claims 2 to 6,
wherein the electrode unit comprises an electrode, and
at least a portion of the electrode is exposed into the inside of the sealed unit.

8. The plasma treatment apparatus of claim 7,
wherein the electrode at least partially exposed into the inside of the sealed unit, is electrically connected to an object to be treated accommodated in the sealed unit or a portion of a container having accommodated therein the object to be treated.

9. The plasma treatment apparatus of any one of claims 2 to 8,
wherein the pressure adjusting unit comprises an exhaust unit for exhausting the inside of the sealed unit, and
the control unit controls the exhaust unit to control the internal pressure of the sealed unit, such that the inside of the sealed unit is sealed against the external environment.

10. The plasma treatment apparatus of any one of claims 2 to 9,
wherein the control unit controls the pressure adjusting unit to maintain the internal pressure of the sealed unit constant within the pre-set process pressure range, and
discharges low-pressure atmosphere inside the sealed unit in which the pressure of the low-pressure atmosphere is maintained constant.

11. The plasma treatment apparatus of claim 10,
wherein the pressure adjusting unit comprises a vacuum pump for exhausting the internal atmosphere of the sealed unit and a valve for opening and closing a flow path interconnecting the sealed unit and the vacuum pump, and
the control unit maintains the internal pressure of the sealed unit constant by opening and closing the valve and discharges plasma inside the sealed unit in which the pressure of the low-pressure atmosphere is maintained constant.

12. The plasma treatment apparatus of claim 10 or 11,
wherein the pre-set process pressure range is set in a range of 2 Torr or higher and less than 30 Torr.

13. The plasma treatment apparatus of any one of claims 2 to 8,
wherein the control unit controls the pressure adjusting unit to change the internal pressure of the sealed unit to be within the pre-set process pressure range, and
discharges low-pressure atmosphere inside the sealed unit in which the pressure of the low-pressure atmosphere is changed.

14. The plasma treatment apparatus of claim 13,
wherein the pressure adjusting unit comprises an exhaust unit for exhausting the internal atmosphere of the sealed unit; a venting unit for injecting external air into the sealed unit; and a valve for opening and closing a flow path interconnecting the exhaust unit or the venting unit and the sealed unit, and
the control unit changes the internal pressure of the sealed unit by opening and closing the valve and discharges plasma inside the sealed unit in which the pressure of the low-pressure atmosphere is changed.

15. The plasma treatment apparatus of any one of claims 2 to 8,
wherein the pressure adjusting unit comprises an exhaust unit for exhausting the inside of the sealed unit, and
the control unit controls the exhaust unit to remove by-products, which are generated by discharging the low-pressure atmosphere inside the sealed unit, from the inside of the sealed unit.
